(19) Europäisches Patentamt
European Patent Office
Office européen des brevets

(11) Veröffentlichungsnummer: **0 190 676 B1**

# (12) EUROPÄISCHE PATENTSCHRIFT

(45) Veröffentlichungstag der Patentschrift: **22.03.89**

(21) Anmeldenummer: **86101269.8**

(22) Anmeldetag: **31.01.86**

(51) Int. Cl.⁴: **C 07 C 91/14**, C 07 C 91/26, C 07 C 101/14, C 07 C 103/44, C 07 F 9/38, A 61 K 31/13, A 61 K 31/16, A 61 K 31/195, A 61 K 31/66

(54) **All-cis-1,3,5-Triamino2,4,6-cyclohexantriol-Derivate, ihre Verwendung, Verfahren zu ihrer Herstellung und sie enthaltende pharmazeutische Präparate.**

(30) Priorität: **02.02.85 DE 3503614**

(43) Veröffentlichungstag der Anmeldung: **13.08.86 Patentblatt 86/33**

(45) Bekanntmachung des Hinweises auf die Patenterteilung: **22.03.89 Patentblatt 89/12**

(84) Benannte Vertragsstaaten: **AT BE CH DE FR GB IT LI LU NL SE**

(56) Entgegenhaltungen:
**FR-A- 1 522 204**
**FR-A- 2 322 143**
**US-A- 3 496 196**

(73) Patentinhaber: **Laboratorien Hausmann AG, Rechenstrasse 37, CH-9001 St. Gallen (CH)**

(72) Erfinder: **Schneider, Walter, Prof. Dr. Anorganische Chemie, Eidgenössische Tech. Hochschule Zürich ETH-Zentrum, Universitätsstrasse 6 CH-8092 Zürich (CH)**
Erfinder: **Erni, Isidor, Dr. sc.nat. Anorganische Chemie, Eidgenössische Tech. Hochschule Zürich ETH-Zentrum, Universitätsstrasse 6 CH-8092 Zürich (CH)**
Erfinder: **Hegetschweiler, Hans K., Dr. sc.nat. Anorganische, Eidgenössische Tech. Hochschule Zürich ETH-Zentrum, Universitätsstrasse 6 CH-8092 Zürich (CH)**

(74) Vertreter: **Türk, Dietmar, Dr. rer. nat. et al, Türk, Gille + Hrabal Patentanwälte Bruckner Strasse 20, D-4000 Düsseldorf 13 (DE)**

EP 0 190 676 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue all-cis-1,3,5-Triamino-2,4,6-cyclohexantriol-Derivate, die auch als cis-1,3,5-Triamino-1,3,5-trideoxy-inositol bezeichnet werden, Verfahren zu ihrer Herstellung, pharmazeutische Präparate, die diese Derivate und/oder den bekannten Grundkörper, das all-cis-1,3,5-Triamino-2,4,6-cyclohexantriol, enthalten, und die Verwendung dieser Derivate und des Grundkörpers zur Herstellung von Arzneimitteln für die therapeutische Anwendung bei Eisenüberladungen im tierischen und insbesondere menschlichen Körper. Die Erfindung betrifft auch die Verwendung der oben genannten Derivate (Liganden) zum Auflösen sonstiger unerwünschter Eisenablagerungen. Ferner betrifft die Erfindung auch die Verwendung der oben genannten Liganden für andere Metallionen, insbesondere als Ionen-Carrier in Ionen-selektiven Elektroden (vgl. «Ion-selective electrodes», Cambridge University Press, Cambridge, [1983]) oder in therapeutischen oder diagnostischen Anwendungen (vgl. Inorganic Chemistry in Biology and Medicine, ACS Symposium Series 140, American Chemical Society, Washington, D.C. [1980] S. 121 bis 140, 91 bis 101 und 103 bis 119).

Eisen ist in biologischen Systemen weit verbreitet und nimmt an wichtigen biochemischen Reaktionen teil. Der Körper eines gesunden, erwachsenen Menschen enthält etwa 4 g Eisen. Mehr als die Hälfte davon (etwa 2,6 g) liegt im Hämoglobin in den roten Blutkörperchen vor. Es gibt verschiedene Störungen des menschlichen Eisenmetabolismus. Eisenmangel ist relativ weit verbreitet, doch sind schwere gesundheitliche Schädigungen selten. Eisenüberladung wird wesentlich seltener beobachtet, doch sind die Auswirkungen auf die Gesundheit bedeutend schwerer. Die einzige Möglichkeit des Körpers, Eisen auszuscheiden, besteht in der Abstoßung von Zellen (Hautschuppen, abgeschilferte Zellen der Darmwand) und in Blutverlust. Übermäßig aufgenommenes Eisen kann zuerst in speziellen Depots gespeichert werden. Wird die Kapazität überschritten, beginnt sich Eisen toxisch auszuwirken. Den so erreichten krankhaften Zustand nennt man Hämochromatose bzw. Hämosiderose. Beispielsweise kann diese verursacht sein durch eine gestörte Regulierung der Eisenaufnahme in Form einer übermäßigen Resorption im vorgerückten Alter (über etwa 40). Ein anderes Beispiel sind gewisse Blukrankheiten, die bisher nur durch ständige Bluttransfusionen bekämpft werden können, was ebenfalls zu einer Eisenüberladung führt. Als Beispiel sei die β-Thalassämie erwähnt (vgl. Inorganic Chemistry in Biology and Medicine, ACS Symposium Series 140, American Chemical Society, Washington, D.C. [1980], S. 251 bis 261). Das dauernd zugeführte Eisen wird als unlösliches Eisen(III)hydroxid (bzw. Eisenoxid-hydroxid, Eisenoxid, Rost) in verschiedenen Organen abgelagert. Solche Eisenhydroxid-Ablagerungen können schon im Alter von 20 bis 25 Jahren zum Tod führen.

Diese Eisenhydroxid-Ablagerungen können durch Verabreichung von Komplexbildnern (Liganden) verzögert werden, die das Eisen in löslicher Form aus dem Körper auszuscheiden vermögen. Es ist bekannt, zu diesem Zweck 30-Amino-3,14,25-trihydroxy-3,9,14,20, 25-pentaaza-2,10,13,21,24-triacontanpentaonmethansulfonat-(Deferoxamin) zu verabreichen (vgl. FR 694 M und Inorganic Chemistry in Biology and Medicine, ACS Symposium Series 140, American Chemical Society, Washington, D.C. [1980], S. 279 bis 312).

Dies hat aber folgende Nachteile: Schon bestehende Ablagerungen werden kaum aufgelöst; es muß parenteral verabreicht werden; die Halbwertzeit im Körper ist sehr kurz, deshalb muß kontinuierlich injiziert werden; die Verbindungen sind sehr schwer zugänglich; es gibt Indikationen für gravierende Nebenwirkungen bei langfristiger Applikation (Sehstörungen).

Der vorliegenden Erfindung liegt deshalb die Aufgabenstellung zugrunde, Liganden zu finden, die möglichst weitgehend folgende Bedingungen erfüllen sollten:

1. Hohe Selektivität für Fe(III), um Nebenwirkungen zu minimalisieren und genügend stabile Komplexe im physiologischen Milieu zu bilden, um die Ausfällung von Eisenhydroxiden zu verhindern.

2. Hinreichend rasches Auflösevermögen für Eisenhydroxide.

3. Geringe Toxizität von Ligand und Komplex.

4. Abbau von Ligand und Komplex hinreichend langsam im Körper, um die Ausscheidung zu ermöglichen.

5. Orale Aufnahme möglich.

In der neueren Literatur wurden die allgemeinen Kriterien zur Erfüllung von Bedingung 1 eingehend erörtert (vgl. Inorganic Chemistry in Biology and Medicine, ACS Symposium Series 140, American Chemical Society, Washington, D.C. [1980], S. 279 bis 312). Es wurde nun gefunden, daß die nachfolgend definierten Cyclohexanderivate sowohl Bedingung 1 wie 2 optimal und 3 bis 5 in hohem Maße erfüllen. Sie sind in vorzüglicher Weise geeignet, Eisen und andere Metalle durch Komplexbildung in Lösung zu bringen und insbesondere aus dem menschlichen und tierischen Körper auszuscheiden.

Entscheidend für die gute Komplexbildung sind

a) All-axial Stellung der Sauerstoffatome am Cyclohexanring, so daß optimale $O_6$-Koordination des Eisens durch zwei bzw. einen durch funktionelle Gruppen substituierten Liganden möglich ist. Die all-axial Stellung wird erzwungen durch drei Ammoniumgruppen (alkyliert oder protoniert).

b) Die Acidifierung der Hydroxylgruppen um zirka 8 pK-Einheiten gegenüber gewöhnlichen aliphatischen Alkoholen durch die positiven Ladungen der quaternisierten bzw. im Neutralbereich protonisierten Stickstoffatome am Cyclohexanring.

Wenn diese beiden Bedingungen nicht erfüllt sind, ergeben drei- oder mehrwertige Alkohole keine stabilen Komplexe mit Eisen im Neutralbereich.

Gegenstand der Erfindung sind deshalb die in den Ansprüchen definierten neuen Verbindungen.

Diese haben, wie dem Chemiker bekannt, nur solche Kombinationen von Substituenten, die aus sterischen Gründen vorhanden sein können.

Das Grundgerüst dieser neuen Verbindungen, das all-cis-1,3,5-Triamino-2,4,6-cyclohexantriol ist bekannt. Die Synthese dieser Verbindung durch Hydrierung von 1,3,5-Triaminophloroglucin (2) wurde mehrmals beschrieben (Quadbeck G., Röhm E., Chem. Ber. 89, 1645–1648 [1956]; Lichtenthaler F.W., Leinert H., Chem. Ber. 99, 903–907 [1966]; Bracher G., Diplomarbeit ETH Zürich [1973]). Die Autoren stellten zuerst das unbeständige und explosive Trinitrosophloroglucin her, welches anschließend mit rauchender Salpetersäure zu Trinitrophloroglucin oxidiert wurde. Diese Reaktion kann nur in Gramm-Mengen durchgeführt werden und ist deshalb für größere Ansätze ungeeignet. Die direkte Nitrierung von Phloroglucin wurde erst in jüngster Zeit publiziert (DeFusco A.A., Nielson A.T., Atkins R.L., Org. Prep. Proced. Int. 14, 393–424 [1982]); es ist eine heikle Reaktion. Eine pharmazeutische Verwendung oder Brauchbarkeit dieses Grundkörpers ist jedoch nicht bekannt. Da die bekannten Herstellungsverfahren, wie erwähnt, sehr unbefriedigend sind, liegt der vorliegenden Erfindung auch die Aufgabe zugrunde, neue und verbesserte bzw. vereinfachte Verfahren zur Herstellung dieser Verbindung und der Derivate hiervon zu finden. Gegenstand der Erfindung ist demgemäß auch das in den Ansprüchen definierte Herstellungsverfahren.

Die neuen Verbindungen gemäß der Erfindung haben eine relativ geringe Toxizität und können intravenös oder in manchen Fällen auch oral verabreicht werden.

Auch das oben erwähnte bekannte Grundgerüst der neuen Verbindungen gemäß der Erfindung, das bisher als Pharmazeutikum nicht empfohlen wurde, besitzt eine relativ geringe Toxizität und ebenfalls eine gute Wirkung, Eisen durch Komplexbildung in Lösung zu bringen.

Gegenstand der Erfindung sind demgemäß auch pharmazeutische Präparate, die das all-cis-1,3,5-Triamino-2,4,6-cyclohexantriol oder Verbindungen der Formeln I bis VI gemäß Anspruch 1 als aktiven Bestandteil, gegebenenfalls zusammen mit üblichen pharmazeutischen Verdünnungsmitteln, Zusatzstoffen oder Exzipien enthalten.

Experimentell hat es sich gezeigt, daß ein β-FeO(OH)-Korn von 50×6×6 nm durch die erfindungsgemäße Verbindung der allgemeinen Formel I mit $R_1=R_2=R_3=R_4=R_5=R_6=CH_3$ fünfmal schneller aufgelöst wird als durch das bekannte Deferoxamin.

Die gemäß der Erfindung verwendeten Verbindungen werden zweckmäßig pro Dosis in einer Menge von 1 bis 50 mg aktivem Bestandteil pro Kilogramm Körpergewicht eingesetzt. Für die chronische Detoxifikation genügen 1–10 mg/kg, für eine akute Detoxifikation können bis zu 50 mg/kg gegeben werden.

Es ist zweckmäßig, daß pro Tag (oder pro Woche) 1 bis 3 Verabreichungen erfolgen.

Die Verbindungen können je nach Bedarf in üblicher Weise zu pharmazeutisch brauchbaren Verabreichungsformen formuliert werden.

1,3,5-Triamino-2,4,6-cyclohexantriole können in zehn verschiedenen diastereomeren Formen bezüglich der Stellung der Stickstoff- und Sauerstoffatome vorliegen. Erfindungsgemäß ist das sogenannte all-cis-1,3,5-Triamino bzw. -Trialkylamino-2,4,6-cyclohexantriol der anspruchsgemäßen Formel von Interesse, die wie folgt vereinfacht dargestellt werden kann.

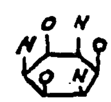

Erfindungsgemäß gelang es, die gewünschten all-cis-1,3,5-Triamino-2,4,6-cyclohexantriole nach folgendem Reaktionsschema herzustellen, wodurch die eingangs aufgezeigten bisherigen Synthesenachteile ausgeräumt werden konnten.

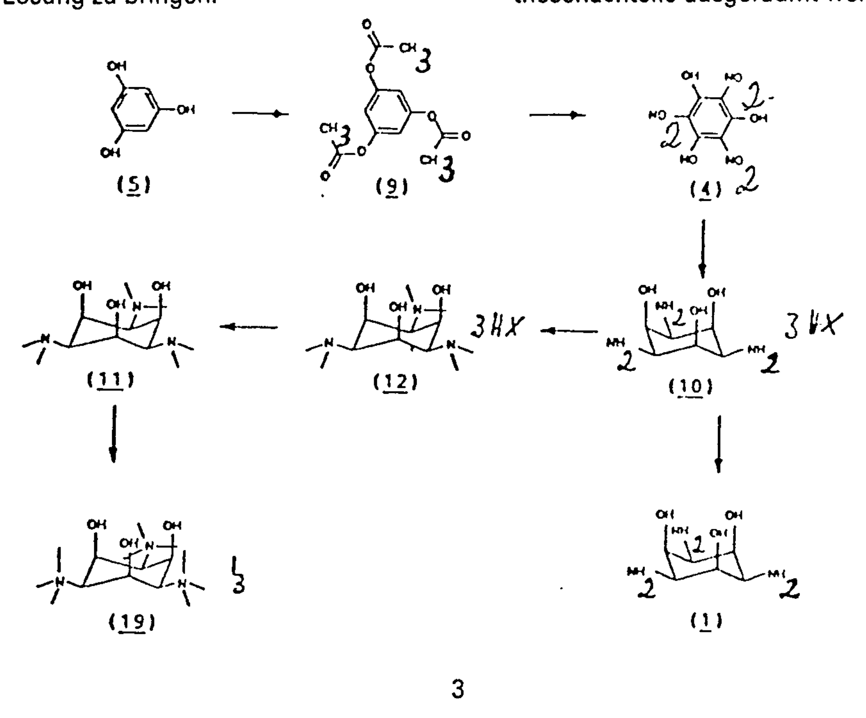

(5) (9) (4) (11) (12) (10) (19) (1)

Die Erfindung betrifft somit ein Herstellungsverfahren, bei dem Phloroglucin (5) mit Acetanhydrid praktisch quantitativ nach der Methode von Heller, Berichte 45, 421 (1912), in das Triacetat (9) umgewandelt wird. So geschützt kann man die Nitrierung nach Nietzki und Moll, Berichte 26, 2185–2187 (1893), in rauchender Salpetersäure gefahrlos durchführen. Das anfallende, mäßig wasserlösliche Trikaliumsalz kann z.B. als schwerlösliches Bariumsalz gefällt und in dieser Form problemlos in reiner Form erhalten werden. Die Zugabe einer stöchiometrischen Menge Säure, z.B. Schwefelsäure setzt dann das gewünschte Trinitrophloroglucin (4) frei.

Das Trinitrophloroglucin wird anschließend hydriert. Dadurch kann man auf die bisher notwendige Isolierung des äußerst sauerstoffempfindlichen Triaminphloroglucines ohne Einbuße an Reinheit und Ausbeute verzichten. An Stelle des freien Phenols (4) kann auch das Monoalkalimetallsalz, insbesondere das Kaliumsalz, verwendet werden.

Die Hydrierung wird unter intensivem, Turbulenz erzeugendem Rühren durchgeführt. Man erhält dabei ein Gemisch verschiedener Polyhydroxypolyamino-cyclohexane aus dem das gewünschte all-cis-1,3,5-Triamino-2,4,6-cyclohexantriol als Salz, z.B. als Schwefelsäuresalz (10a) durch wiederholtes Umkristallisieren, z.B. aus Wasser-Methanol, isoliert werden kann.

Verschiedene Salze lassen sich beispielsweise durch Ionentausch an einem Anionentauscherharz aus dem primär erhaltenen Sulfat (10a) darstellen, z.B. das Trichlorid (10b), das Triformiat (10c) oder auch die freie Base (1). Alternativ kann eine Stöchiometrische Umsetzung des Sulfats (10a) mit Bariumhydroxid erfolgen, die ebenfalls zu (1) führt.

Zur Herstellung der N-alkylierten oder N-acylierten Verbindungen kann das freie Amin oder sein Salz alkyliert bzw. acyliert werden. Es können die üblichen zur Alkylierung bzw. Acylierung von Aminen geeigneten Mittel verwendet werden.

Beispiele für Alkylierungsmittel sind Alkylhalogenide (beispielsweise Bromide und Jodide) mit 1 bis 18 Kohlenstoffatomen (z.B. Methyl-, Ethyl-, n-Propyl-, Isopropyl-, n-Butyl-, sec.-Butyl- und tert-Butyljodid oder -bromid). Beispiele für Olefine und Alkohole, die durch Addition bzw. Kondensation zur Alkylierung verwendet werden können, sind Olefine mit 1 bis 18 Kohlenstoffatomen (z.B. solche mit aktivierenden Substituenten, wie Nitrilfunktionen, z.B. Acrylnitil) und Alkohole mit 1 bis 18 Kohlenatomen (z.B. solche mit aktivierenden Substituenten wie Nitrilfunktion, z.B. Glykolsäurenitril). Es ist auch eine reduktive Alkylierung mit Aldehyden und Ketonen mit 1 bis 18 Kohlenstoffatomen möglich.

Beispiele für Acylierungsmittel sind reaktionsfähige Derivate von Alkansäuren mit 1 bis 18 Kohlenstoffatomen im Alkylteil, wie Säurehalogenide, insbesondere Säurechloride, Säureanhydride und Ester. Spezielle Beispiele sind Acetylchlorid, Propionylchlorid, Caprylsäurechlorid und Bernsteinsäureanhydrid.

Die Alkylierungs- und Acylierungsmittel können durch eine oder mehrere gleiche oder verschiedene funktionelle Gruppen oder deren Vorläufer substituiert sein, insbesondere solche, die an Metallkationen, insbesondere Eisen(III), koordinieren können. Derartige funktionelle Gruppen können in geschützter Form vorliegen, insbesondere dann, wenn die Gefahr besteht, daß sie unter den gewählten Alkylierungs- oder Acylierungsbedingungen beeinflußt werden oder in die Reaktion eingreifen können.

Beispiele für derartige Substituenten sind: Hydroxylgruppen, Carboxylgruppen (und ihre Derivate, wie Salze, Amide und Ester sowie die Nitrilgruppe als ihre Vorläufer), $-CON(OH)R$, worin R für eine Alkylgruppe mit 1 bis 6, insbesondere 1 bis 4 Kohlenstoffatomen steht, $-OPO_3H$ oder deren Salze und Ester, $-PO_3H$ oder deren Salze und Ester, $-SR$, worin R wie für $-CON(OH)R$ definiert ist, und $-CN$, sowie

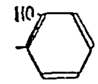

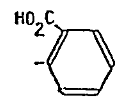

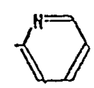

und/oder

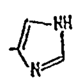

und/oder deren Salze.

Zur Herstellung der Dietherderivate der allgemeinen Formel III können die all-cis-1,3,5-Triamino-2,4,6-trihydroxycyclohexan-Verbindungen in Form ihrer quaternisierten Derivate (beispielsweise der Formel II) mit den entsprechenden difunktionellen Alkylierungsmitteln, z.B. Alkylenhalogeniden, z.B. Methylen- oder Ethylenhalogeniden (z.B. Bromiden oder Jodiden) umgesetzt werden. Man arbeitet vorzugsweise in Anwesenheit von starken Basen, wie Alkoholaten, beispielsweise Alkalimetall- und/oder Erdalkalimetallmethoxid oder -ethoxid in dem entsprechenden Alkohol als Lösungsmittel.

Die Herstellung der Diaminderivate der allgemeinen Formel IV erfolgt durch Alkylierung der all-cis-1,3,5-Triamino-2,4,6-trihydroxycyclohexan-Derivate der allgemeinen Formel I mit einem difunktionellen Alkylierungsmittel, z.B. Alkylenhalogeniden mit 4 bis 8 Methylengruppen. Statt der Amine der Formel I kann auch von Komplexen aus 2 Molekülen davon mit Eisen(III) oder Chrom(III) ausgegangen werden.

Die Alkylierung der freien Amine bzw. deren Salze führt bei der Verwendung von Alkylierungsmitteln mit kurzkettigen Alkylresten, insbesondere dem Methylrest, im allgemeinen zu sekundären

Aminen; sie kann bis zur Quaternisierung weitergeführt werden. Bei der Verwendung von Alkylierungsmitteln zur Einführung längerkettiger, sekundärer oder tertiärer Alkylgruppen sowie zur Einführung von Alkylgruppen mit sperrigen Substituenten, erhält man im allgemeinen die entsprechenden primären Aminderivate.

Zur Herstellung der Tris-(dialkylamino)-Verbindungen (11) ist die reduktive Alkylierung mit Aldehyden und Ketonen besonders gut geeignet. Zur Herstellung der Tris-(dialkylamino)-Verbindungen (11) kann z.B. das Salz 10 (beispielsweise das Sulfat, Trichlorid oder Triformiat) alkyliert werden. Es kommen beispielsweise folgende Alkylierungsmethoden in Betracht:

a) Die reduktive Alkylierung mit Aldehyden oder Ketonen (z.B. Formaldehyd, Acetaldehyd, Propionaldehyd, Aceton) und Ameisensäure nach Leuckart-Wallach. Das gewünschte Amin (12) läßt sich als Hydrochlorid in Ausbeuten bis zu 50% durch mehrmalige Umkristallisation aus z.B. Alkohol (z.B. Methanol) isolieren.

b) Die reduktive Alkylierung mit wäßriger Aldehyd- oder Ketonlösung (z.B. Formaldehyd-, Acetaldehyd-, Propionaldehyd-, Aceton-Lösung) und Platin/Wasserstoff als Reduktionsmittel. Diese Reaktion liefert ausschließlich das gewünschte Stereoisomer (11) in guter Ausbeute. Beispielsweise all-cis-1,3,5-Tris-(dimethylamino)-2,4,6-cyclohexantriol wurde jeweils als Hydrochlorid (12a) oder als Sulfat (12b) isoliert. Durch Ionentausch gegen Hydroxid konnte das freie Triamin (11) als Monohydrat erhalten werden. In dieser Form ist die Löslichkeit in vielen organischen Lösungsmitteln gering. Kochen am Rückfluß in Hexan ergibt das wasserfreie Produkt, welches vermutlich aufgrund besonderer struktureller Eigenheiten (Wasserstoffbrücken) in allen üblichen Lösungsmitteln gut löslich ist.

Die erhaltenen alkylierten Produkte können durch Alkylieren quaternisiert werden. Beispielsweise führt die Umsetzung mit Alkylhalogeniden z.B. Methyl-, Ethyl-, Propyl-, Isopropyl-, Butyl-Halogeniden (z.B. Jodiden, Bromiden und Chloriden) nach folgendem Schema zu den entsprechenden Mono-, di- und triquaternären Produkten.

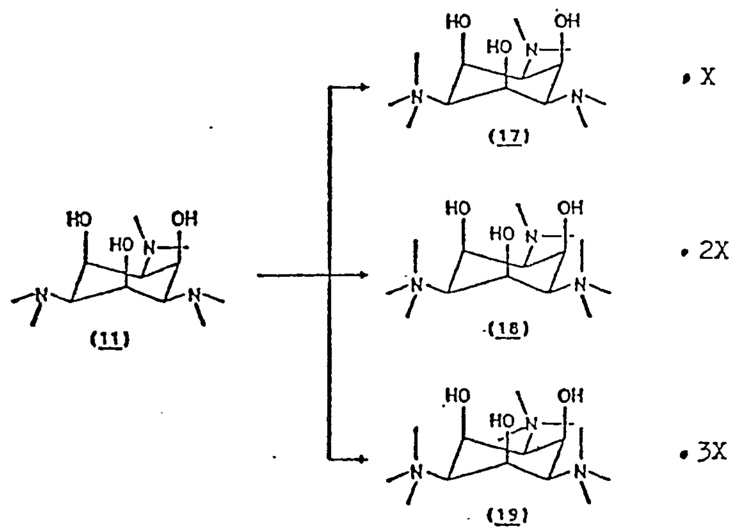

(11) (17) • X (18) • 2X (19) • 3X

(19a): X = I
(19b): X = $NO_3$
(19c): X = Cl

Beispielsweise konnte mit Methyljodid als Quaternisierungmsittel all-cis-1,3,5-Tris-(dimethylamino)-2,4,6-cyclohexantriol (11) zu den di- und triquartären Produkten (18) und 19a) umgesetzt werden. Es gelang auch die monoquartäre Verbindung (17) zu isolieren.

Aus den anfallenden Gemischen läßt sich das triquartäre Iodid (19a) durch Umkristallisation z.B. aus schwach alkalischer wäßriger Lösung von (17) und (18) abtrennen.

Eine Triquaternisierung ist z.B. auch in alkalischer wäßriger Lösung von all-cis,1,3,5-Tris-(dimethylamino)-2,4,6-cyclohexantriol mit einem Überschuß von Bromhydrinen oder Epoxiden (z.B. Bromethanol oder Ethylenoxid) möglich. Dabei wird bevorzugt der pH-Wert durch Laugenzugabe auf einen Wert auf etwa 10 reguliert.

Die erfindungsgemäßen Verbindungen, insbesondere diejenigen, worin $R_1$–$R_6$ bzw. $R_7$–$R_9$ die Bedeutung von –CO-Alkyl haben, können auch nach der Verfahrensweise von Lichtenthaler F.W., Leinert H., Chem. Ber. 99, 903–907 (1966) hergestellt werden (dort ist die Verbindung der allgemeinen Formel I beschrieben, worin $R_1=R_3=R_5=-COCH_3$ und $R_2=R_4=R_6=H$).

Geeignete pharmazeutisch brauchbare Salze der erfindungsgemäßen Verbindungen sind solche mit einer organischen Säure (z.B. ein Acetat, Maleat, Tartrat, Methansulfonat, Benzolsulfonat, Formiat, Toluolsulfonat) oder mit einer anorganischen Säure (z.B. Chlorid, Bromid, Sulfat, Phosphat).

Die erfindungsgemäßen, eine oder mehrere der erfindungsgemäßen Verbindungen enthaltenden pharmazeutischen Zusammensetzungen können beispielsweise fest oder flüssig sein und in pharmazeutischen Formen vorliegen, wie sie üblicherweise in der Humanmedizin oder Tiermedizin verwendet werden, wie z.B. als einfache oder dragierte Tabletten, Gelbkapseln, Kapseln, Granulate,

Suppositorien und injizierbare Präparate. Sie werden nach üblichen Methoden hergestellt. Der oder die Wirkstoffe können hierbei in derartigen pharmazeutischen Zusammensetzungen üblicherweise verwendete Exzipienten eingebracht werden, wie Talk, Gummi arabicum, Lactose, Stärke, Magnesiumstearat, Kakaobutter, wäßrige oder nichtwäßrige Träger, Fettkörper tierischen oder pflanzlichen Ursprungs, Paraffinderivate, Glykole, verschiedene Netz-, Dispergier- oder Emulgiermittel und Konservierungsmittel.

Beispiele für Metallionen, für die, wie oben erwähnt, die Verbindungen nach Anspruch 1 und/oder das all-cis,1,3,5-Triamino-2,4,6-cyclohexantriol eingesetzt werden können, sind Erdalkaliionen, insbesondere Magnesium, aber auch Calcium, Strontium, Barium. Andere Beispiele sind die Ionen von seltenen Erden, insbesondere Gadolinium. Eine andere Gruppe von Ionen sind diejenigen von Aluminium, Gallium, Indium oder Technetium. Die gemäß der Erfindung verwendeten Liganden bilden mit diesen Metallen besonders leicht und besonders beständige und/oder besonders gut lösliche Komplexverbindungen. Andererseits sind die vorgenannten Metalle von großer Bedeutung auf den oben erwähnten Anwendungsgebieten.

Beispiel 1
Synthese von all-cis-1,3,5,Tris-(dimethylamino)-cyclohexantriol

A) Herstellung der Ausgangsmaterialien:
1. Phloroglucintriacetat (Heller 1912)

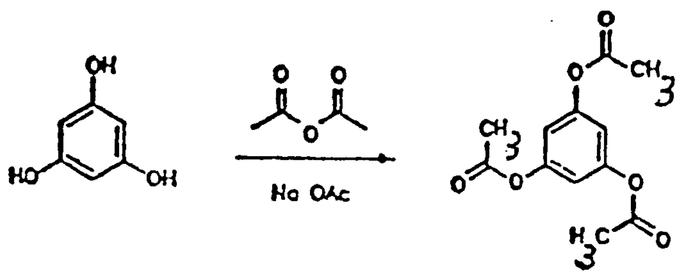


$C_6H_6O_3$
126.11

$C_{12}H_{12}O_6$
252.23

Edukte:
– Phloroglucin, Fluka puriss., wasserfrei, 12 h im Trockenschrank bei 100°C getrocknet.
– Natriumacetat, wasserfrei, 12 h im Trockenschrank bei 160°C getrocknet.
300 g (2.85 Mol) Phloroglucin und 300 g (3.66 Mol) Natriumacetat werden in einem 3 l-Rundkolben, ausgestattet mit Magnetrührer und Dimrothrückflusskühler, in 1.7 l (18 Mol) Acetanhydrid suspendiert. Die Aufschlämmung wird mit einem Siliconölbad auf 130°C erwärmt. Man rührt während zwei Stunden bei dieser Temperatur und läßt dann abkühlen. Der Inhalt, der zu einem weißen Brei ertstarrt ist, wird auf 10 kg Eis gegossen. Man rührt die Suspension mit einem Glasstab mehrmals kräftig um und nutscht anschließend ab. Der Festkörper wird zweimal gründlich gewaschen, indem man ihn in einem 5 l-Becherglas mit 2 l Wasser suspendiert, mit einem Glasstab gut umrührt und anschließend abnutscht.

Das Rohprodukt wird zweimal umkristallisiert: Man löst in 2 l siedendem Ethanol, läßt während mindestens 12 h bei 0°C stehen und nutscht ab. Das gereinigte Produkt läßt man während einem Tag bei 0.01 torr und Raumtemperatur trocknen. Die Ausbeute beträgt mehr als 570 g (2.26 Mol=95%). Aus der Mutterlauge kann noch weiteres Produkt gewonnen werden. So erhaltenes Phloroglucinacetat schmilzt bei 106°C (Literaturwert: 105 – 106 °C)

2. tri-Barium-bis-(trinitrophloroglucinat)
Nietzki 1893, Benedikt 1878)

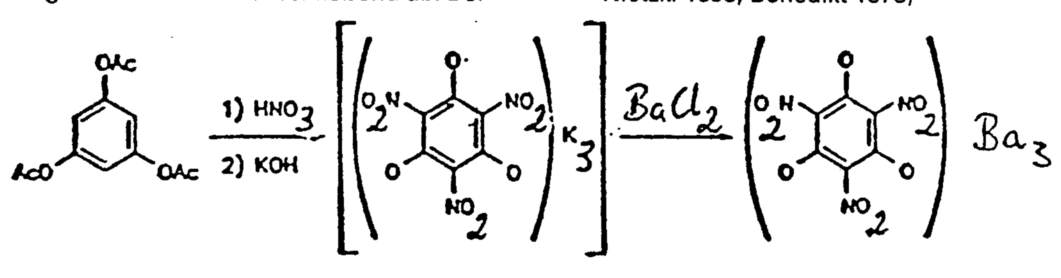


$C_{12}H_{12}O_6$
252.23

$(C_6N_3O_9)K_3$
375.38

$C_6N_3O_9)_2Ba_3$
928.18

In einer Kapelle wird hinter einer Schutzscheibe ein 1 l-Becherglas mit einem Magnetrührer, einem Thermometer und einem Kühlbad ausgestattet. Darin kühlt man 400 ml (600 g, 9,5 Mol) rauchende Salpetersäure (Salpetersäure rauchend 100 %, Dichte etwa 1.52) mit einer Eis-Kochsalz-Kühlmischung auf 0°C ab. Man sorgt weiter für die gute Kühlung und trägt 100 g (0.4 Mol) feingepulvertes Phloroglucintriacetat in kleinen Portionen so ein, daß die Temperatur nie über 15 – 20 °C steigt.

Während der Zugabe, die etwa 2 Stunden dauert, entweichen nitrose Gase.

Anschließend wird die klare, braunrote Lösung in ein 3l-Becherglas auf 1 kg Eis gegossen. Dabei fällt ein gelbgrüner Festkörper (Trinitrophloroglucintriacetat) aus. Die Suspension wird nun unter Kühlung und Rühren mit einem Glasstab mit 50% KOH-Lösung (1.3 kg, entsprechend 650 g [11.6 Mol] KOH in 650 ml Wasser) versetzt, bis die Farbe von goldgelb auf tief rotbraun umschlägt und stark alkalische Reaktion (ph > 10) festgestellt werden kann. Während der Neutralisation steigt die Temperatur bis auf ca 60 °C an, und unter Aufschäumen bilden sich nitrose Gase.

Die ca. 20 °C kalte Suspension, welche schwach nach Ammoniak riecht, wird durch eine Glasfilternutsche (F4) filtriert. Das Filtrat wird verworfen, der Festkörper, bestehend aus Trikaliumtrinitrophloroglucinat und Kaliumnitrat, in einem 5 l-Rundkolben in 3 l heißem Wasser gelöst. Zur heißen Lösung tropft man unter intensivem Rühren mit einem Magnetrührer eine Lösung von 250 g (1.2 Mol) Bariumchlorid ($BaCl_2 \cdot 2\ H_2O$) in 1 l Wasser. Die Zugabe dauert 5 – 10 Minuten. Dabei fällt ein microkristalliner, gelber Festkörper aus. Die Suspension wird während 10 Minuten weiter gerührt und anschließend abgenutscht. Man suspendiert den Rückstand in 2 l Wasser, rührt während 15 Minuten und nutscht erneut ab. Das Produkt wird mit Ethanol gewaschen und am HV bei 0.01 Torr und Raumtemperatur getrocknet. Das Trocknen bis zur Gewichtskonstanz dauert ca. 24 h. Die Ausbeute beträgt 100 – 125 g (0.11 – 0.13 Mol) Bariumsalz.

3. Trinitrophloroglucin
(Nietzki 1893, Benedikt 1878)

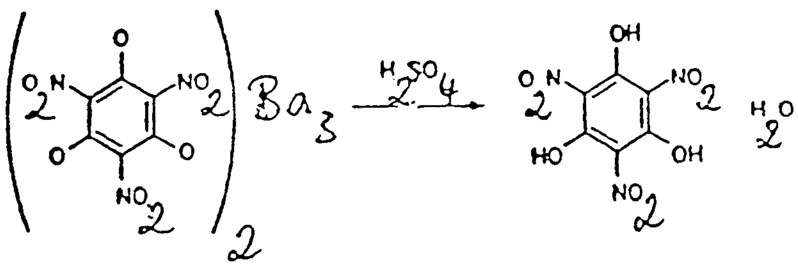

$(C_6N_3O_9)_2Ba_3$
928.18

$C_6H_3N_3O_9H_2O$
279.12

100 g (0.11 Mol) Tri-Barium-bis-(trinitrophloroglucinat) werden in einem 5 l-Rundkolben, ausgestattet mit einem Magnetrührer, in 2.5 l Wasser aufgeschlämmt. Unter Rühren versetzt man die Suspension mit 340 ml (0.34) Mol) 1 M wässriger Schwefelsäure. Man läßt mindestens 6 h weiter rühren und filtriert anschließend durch Celite. Durch Auswaschen des Rückstandes kann die Ausbeute noch etwas verbessert werden. Dazu wird das abfiltrierte Bariumsulfat zusammen mit dem Celite in einem Becherglas in 500 ml Wasser suspendiert und während 15 Minuten gerührt. Der Festkörper wird abzentrifugiert und die überstehende Lösung durch eine mit Celite beschichtete Nutsche filtriert. Man schlämmt den Rückstand erneut in Wasser auf und wiederholt das geschilderte Vorgehen so lange, bis die überstehende Lösung nach dem Zentrifugieren praktisch farblos ist (2 – 3mal)
Die vereinten Filtrate werden am Rotationsverdampfer bei einer Badtemperatur von 30 – 40 °C auf 100 ml eingeengt, wobei ein gelber Festkörper ausfällt. Die Suspension wird in einen 1 l-Birnenkolben transferiert und auf dem Wasserbad auf 98 °C erwärmt. Man gibt in kleinen Portionen soviel Wasser zu, bis eben alles gelöst wird, filtriert die heiße Lösung durch eine mit dem Fön vorgewärmte Nutsche und läßt langsam auf 4 °C abkühlen. Dabei kristallisiert Trinitro-phloroglucin-hydrat in großen, gelben Nadeln, die auf einer Nutsche von der Mutterlauge abgetrennt und am Wasserstrahlvakuum bei Raumtemperatur getrocknet werden.
Ausbeute: 60 g (0.21 Mol) Trinitrophloroglucin

Aus der Mutterlauge kann weiteres Produkt gewonnen werden. Die Aufarbeitung lohnt sich jedoch nur für größere Mengen. Man versetzt die Lösung unter Rühren mit 10 M KOH-Lösung bis stark alkalische Reaktion festgestellt werden kann (pH > 13). Dabei fällt festes, goldgelbes Trikaliumtrinitrophloroglucinat aus. Man läßt die Suspension 12 h bei 4 °C stehen und nutscht ab. Der Festkörper wird in heißem, (< 80 °C) Wasser gelöst und bis zur vollständigen Fällung mit Bariumchloridlösung versetzt. Das so erhaltene tri-Barium-bis(trinitrophloroglucinat) wird nach obiger Vorschrift mit wäßriger Schwefelsäure umgesetzt. Man erhält Trinitrophloroglucin von guter Reinheit.

B) all-cis-1,3,5,-Triammonio-2,4,6-cyclohexantriol-sulfat
(Quadbeck 1956, Lichtenthaler 1966)

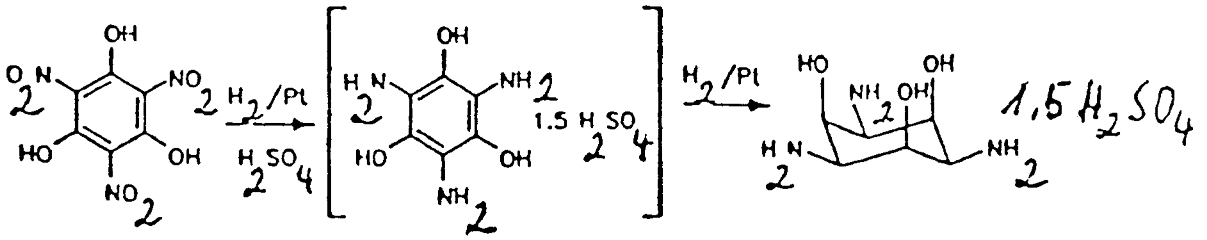

$C_6\ H_3\ N_3\ O_9\ H_2$ 279.12 | $C_6\ H_9\ N_3\ O_3$ 1.5 $H_2SO_4$ 318.27 | $C_6\ H_{15}\ N_3\ O_3$ 1.5 $H_2SO_4$ 324.32

Ca. 4.4 g (18 mMol) frisch hergestelltes Platindioxidhydrat werden in einem 2 l-Glasautoklav mit Kunststoffrührer in 255 ml 1 M wäßriger Schwefelsäure vorgelegt. Unter intensivem Rühren wird bei einem Wasserstoffdruck von 10 Bar vorhydriert. Die Reduktion dauert wenige Minuten. Ihr Ende ist an der Koagulation von schwarzem Platin erkennbar.

Der Autoklav wird nun geöffnet, und man gibt 47 g (168 mMol) festes Trinitrophloroglucin zu. Die Suspension wird unter Kühlung und intensivem Rühren bei einem Wasserstoffdruck von 10 Bar hydriert. Die erste Stufe (Reduktion der Nitrogruppen) verläuft relativ schnell, dabei wird erheblich Wärme freigesetzt. Bei der zweiten Stufe (Hydrierung des Aromaten) ist die Kühlung nicht mehr erforderlich. Das Ende der ersten Stufe erkennt man am Verschwinden der gelben Farbe.

Die Reduktionsdauer hängt stark von der Rührintensität ab. Die angegebene Reaktionsdauer bezieht sich auf ein mechanisch angetriebenes Rührwerk mit einer Rührgeschwindigkeit von 1200 U/min. Mit der Zeit fällt ein weißer Festkörper aus. Nach 10 Tagen wird die Reaktion abgebrochen und die Suspension in einen 5 l-Rundkolben transferiert. Man verdünnt mit Wasser auf ein Volumen von 3.5 l und rührt, bis sich der weiße Festkörper vollständig aufgelöst hat. Der Katalysator wird mit einer G4-Nutsche abgetrennt und das praktisch farblose Filtrat am Rotationsverdampfer auf 500 ml eingeengt. Die Suspension wird mit 1 M Schwefelsäure versetzt, bis sich ein pH-Wert von ca. 2 eingestellt hat. Man gibt 1.2 l Methanol zu, läßt die Suspension mindestens 12 h bei 4 °C stehen und nutscht ab. Das Produkt wird 6mal wie folgt umkristallisiert: Man löst in der minimalen Menge (ca. 1.6 l) Wasser (25 °C), filtriert durch eine G4-Nutsche und versetzt die Lösung unter Rühren langsam mit 750 ml Methanol. Die Suspension wird über Nacht bei 4 °C stehengelassen und der Festkörper mit einer Nutsche von der Mutterlauge abgetrennt. Das Produkt wird zuerst im Trockenschrank bei 80 °C, anschließend am Vakuum bei 0.01 Torr und 80 °C bis zur Gewichtskonstanz getrocknet.

Die Ausbeute beträgt 27 g (84 mMol). Dies entspricht 50% bezüglich eingesetztem Trinitrophloroglucin.

Das benötigte Platindioxidhydrat kann beispielsweise vor der Reaktion jedesmal aus Ammoniumhexachloroplatinat (IV) nach einer modifizierten Org. Syntheses Vorschrift (Adams 1964) hergestellt werden:

$$(NH_4)_2PtCl_6 \xrightarrow{NaNO_3\ (l)} PtO_2 \xrightarrow{H_2O} PtO_2\ H_2$$

443.89 | 227.09 | 245.1

Ein Gemisch von 100 g Natriumnitrat und 8 g Ammoniumhexachloroplatinat wird in ein 250 ml-Pyrexbecherglas eingewogen (die in Org. Syntheses [1964] beschriebene Verwendung von Kaliumnitrat führt während der Reaktion zum Zerspringen des Becherglases). Das Gemisch wird in einer Kapelle in einem offenen Tiegelofen erwärmt. Die Temperatur kann mit einem geeichten Chromel-Alumel-Thermoelement kontrolliert werden (Handbook 1974). Das Gemisch verfärbt sich braun und unter Schmelzen tritt lebhafte Gasentwicklung ein. Schaumbildung und Spritzer können durch Rühren mit einem Glasstab verhindert werden. Man erhält schließlich eine leichtbewegliche Schmelze, die 30 Minuten lang auf 500 bis 540 °C (19.8 – 21.5 mV, Ref.=20 °C) erwärmt wird. Beim anschließenden Abkühlen erstarrt die Flüssigkeit. Der erkaltete Inhalt wird dann mit mindestens 150 ml Wasser versetzt und gerührt bis sich alles Natriumnitrat aufgelöst hat. Der dunkelbraune Festkörper wird mit einer G4-Nutsche von der Nitratlösung abgetrennt und ungetrocknet ohne weitere Charakterisierung verwendet.

Vor und nach dem Kontakt des Pt-Katalysators mit Wasserstoff muß mehrmals mit Stickstoff gespült werden.

Das Reaktionsgemisch darf nicht mit Apparateteilen aus (rostfreiem) Stahl in Berührung kommen, da sonst während der Reaktion das durch Korrosion gelöste Eisen mit dem anfallenden Produkt komplexiert wird. Diese Metallkomplexe können nachher nur schwer abgetrennt werden.

Beispiel 2
all-cis-1,3,5-Tris-(dimethylammonio)-cyclohexantriol-trichlorid-dihydrat

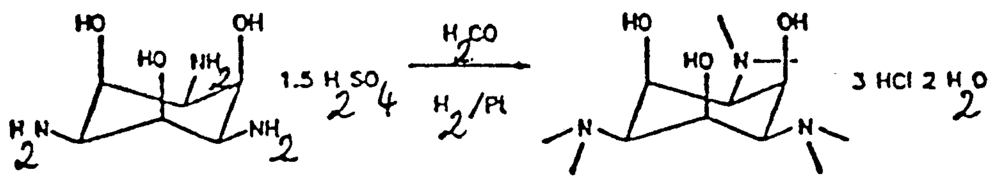


$C_6H_{15}N_3O_3 \cdot 1.5\ H_2SO_4$
324.32

$C_{12}H_{27}N_3O_3 \cdot 3\ HCl \cdot 2\ H_2O$
406.78

Ca. 2.2 g (9.0 mMol) frisch hergestelltes Platindioxidhydrat werden in 200 ml Wasser aufgeschlämmt. Die Suspension wird in einem 2 l-Glasautoklaven 15 Minuten lang bei 10 Bar und Raumtemperatur unter intensiver Rührung (1200U/min) vorhydriert.

Nun öffnet man den Autoklaven und gibt 15 g (46.3 mMol) all-cis-Triammonio-cyclohexantriolsulfat und 40 g (533 mMol) 40% wäßriger Formaldehydlösung zu. Die Hydrierung wird anschließend bei 10 Bar und Raumtemperatur während 24 Stunden fortgesetzt. Dann wird der Autoklav erneut geöffnet und der ph-Wert der Lösung geprüft. Falls deutlich saure Reaktion (pH > 4) festgestellt wird, muß die Hydrierung für 24 Stunden fortgesetzt werden. Andernfalls transferiert man das Reaktionsgemisch in ein 2 l-Becherglas und trennt den Katalysator mit einer G4-Nutsche ab. Das Filtrat wird am Rotationsverdampfer zur Trokkene eingedampft, in 100 ml Wasser gelöst und auf eine Ionentauschersäule Dowex 1, $Cl^-$-Form (Säulenabmessung: Länge 30 cm, Durchmesser 3 cm; Ionenaustauscherharz: Dowex 1, X 4. 50/100 mesh, Chloridform; Aktivierung und Regenierung: mit 2 l 2 M HCl eluiren, anschließend mit Wasser, bis das Eluat neutral reagiert) aufgetragen. Man eluiert, bis das Filtrat chloridfrei ist und prüft mit verdünnter Bariumchloridlösung auf Sulfat. Falls eine Fällung von Bariumsulfat beobachtet werden kann, muß der Ionenaustausch an einer regenerierten Säule wiederholt werden. Die Lösung wird nun am Rotationsverdampfer zur Trockene eingedampft und der farblose Rückstand aus der minimalen Menge siedendem Methanol (ca. 300 ml) umkristallisiert. Aus der Mutterlauge wird weiteres Produkt gewonnen, in dem sie zur Trockene eingedampft und der Rückstand aus siedendem Methanol umkristallisiert wird. Beide Fraktionen werden mit Ether gewaschen und bei 0.01 Torr und Raumtemperatur 12 Std. lang getrocknet. Ausbeute: 12 g (29.5 mMol) = 63.7%.

Beispiel 3
all-cis-1,3,5,-Tris-(dimethylamino)-2,4,6-cyclohexantriol

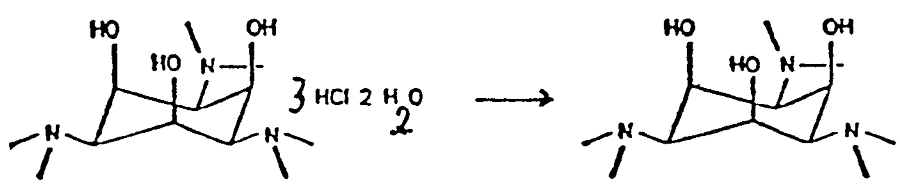


$C_{12}H_{27}N_3O_3\ 3\ HCl\ 2\ H_2O$
406.78

$C_{12}H_{27}N_3O_3$
261.37

12 g (29.5 mMol) Tris-(dimethylammonio)-cyclohexantriol-tri-chlorid-dihydrat werden, in 100 ml Wasser gelöst, auf eine Ionentauschersäule Dowex 1, OH-Form (Säulenabmessung: Länge 30 cm, Durchmesser 3 cm; Ionentauscherharz: Dowex 1, X 4, 50/100 mesh; Aktivierung und Regenerierung: mit 2 l 2 M Salzsäure eluieren; mit Wasser waschen bis das Eluat neutral reagiert; mit 2 l 0.2 M NaOH-Lösung ($CO_2$-freies Wasser verwenden) eluieren. (Eluat muß am Schluß alkalisch reagieren); mit $CO_2$-freiem Wasser eluieren bis das Eluat neutral reagiert), aufgetragen. Man eluiert mit Wasser bis das Eluat neutral reagiert. Nach dem Eindampfen am Rotationsverdampfer erhält man einen weißen Festkörper (all-cis-1,3,-5-Tris-(dimethylamino)-cyclohexantriol-hydrat $C_{12}H_{27}N_3O_3 \cdot H_2O$), der während 30 Minuten in 500 ml Hexan am Rückfluß gekocht wird. Man destilliert anschließend 200 ml Lösungsmittel ab (Azeotrop Wasser-Hexan) und filtriert die heiße Lösung durch einen Papierfilter. Dann werden weitere 150 ml abdestilliert.

Man läßt während 24 h bei 4°C stehen. Dabei kristallisiert ein farbloser Festkörper, der erneut aus 150 ml siedendem Hexan umkristallisiert wird. Das Produkt wird abgenutscht und bei 0.01 Torr und Raumtemperatur bis zur Gewichtskonstanz getrocknet.

Man erhält 7 g (26.8 mMol) wasserfreies Tris-(dimethylamino)-cyclo-hexantriol.

Physikalische Parameter der in den vorstehenden Beispielen erhaltenen Produkte

Phloroglucintriacetat (9)
Smp: 106 °C

Analyse:

| $C_{12}H_{12}O_6$ | C | H |
|---|---|---|
| berechnet | 57.14% | 4.80% |
| gefunden | 57.07% | 4.79% |

$^1$H-NMR (90 MHz, $CDCl_3$):
6.84 ppm (s, 3H), 2.23 ppm (s, 9H).
$^{13}$C-NMR (62.9 MHz, $CDCl_3$, breitbandentkoppelt):
168.4 ppm, 151.1 ppm, 112.7 ppm.

Trinitrophloroglucin (4)
Smp: 167 °C (Zersetzung, vgl. DeFusco [1982])

all-cis-1,3,5-Triammonio-2,4,6-cyclohexantriol-sulfat (10a)
Analyse:

| $C_6H_{15}N_3O_3$ 1.5 $H_2SO_4$ | C | H | N | S |
|---|---|---|---|---|
| berechnet | 22.22 | 5.59 | 12.96 | 14.83 |
| gefunden | 22.22 | 5.69 | 12.80 | 14.91 |

$^1$H-NMR (90 MHz, $D_2O$):
4.8 ppm (s, HDO), 4.5 ppm (m, 3H), 3.8 ppm (m, 3H).
$^{13}$C-NMR (62.9 MHz, $D_2O$, breitbandentkoppelt):
65.6 ppm, 50.5 ppm.

Beispiel 4
all-cis-1,3,5-Triammonio-2-4-6-cyclohexantriol-trichlorid (10b)

Eine wäßrige, gesättigte Lösung des Sulfats (10a) wurde an einem Anionentauscherharz (Dowex 1, C1-Form) chromatographiert. Man engte am RV zur Trockene ein und löste in der minimalen Menge Wasser. Beim Einleiten von Chlorwasserstoff kristallisierte das gewünschte Chlorid (10b). Die Ausbeute war praktisch quantitativ.
$^1$H-NMR (90 MHZ, $D_2O$): identisch mit (10a).

Beispiel 5
all-cis-1,3,5-Triamino-2-4-6-cyclohexantriol (1)

a) Eine wäßrige, gesättigte Lösung von 303 mg (0.93 mMol) Sulfat (10a) wurde auf eine Anionentauschersäule (Dowex 1, OH-Form) gegeben. Man eluierte mit Wasser, bis das Eluat neutral reagierte. Nach dem Einengen am RV erhielt man einen farblosen Festkörper, der aus 50 ml siedendem Ethanol umkristallisiert wurde. Es resultierten 70 mg (0.36 mMol, 38%)Amin.
b) 5.93 g (18.3 mMol) Sulfat (10a) wurden in 1 l Wasser gelöst. Man gab 281 ml $9.76 \cdot 10^{-2}$ M Bariumhydroxidlösung zu, ließ 12 Stunden stehen und filtrierte durch Celite. Die farblose Lösung wurde am RV zur Trockene eingeengt. Man erhielt einen farblosen Festkörper.

Die beiden Produkte konnten anhand von üblichen Charakterisierungsmethoden nicht unterschieden werden:
Smp: ab ca. 150 °C Verfärbung, 200 – 210 °C Verkohlung
(Lichtenthaler, 1966): 203 – 204 °C.

$^1$H-NMR (90 MHz, $D_2O$):
4.8 ppm (s, HDO), 3.83 ppm (m, 3H), 2.80 ppm (m, 3H).
$^{13}$C-NMR (62.9 MHz, $D_2O$, breitbandentkoppelt):
73.7 ppm. 51.7 ppm.

Beispiel 6
all-cis-1,3,5-Triammonio-2-4-6-cyclohexantriol-tri-formiat (10c)

6.75 g (20.8 mMol) Sulfat (10a) wurden in 1 l Wasser gelöst und an einem Anionentauscherharz (Dowex 1, Formiat-Form) chromatographiert. Das Eluat wurde am RV (neigt zu Siedeverzügen) zu einem Öl eingeengt, in wenig Wasser aufgenommen und bis zur Trübung mit Aceton versetzt. Bei 4 °C erhielt man ein farbloses Kristallisat.
Ausbeute: 4.63 g (14.7 mMol, 71%).
Analyse:

| $C_9H_{21}N_3O_9$ | C | H | N |
|---|---|---|---|
| berechnet | 34.29% | 6.71% | 13.33% |
| gefunden | 34.00% | 6.84% | 13.00% |

Physikalische Parameter der in den vorstehenden Beispielen erhaltenen Produkte:
Analyse:
all-cis-1,3,5-Tris(dimethylammonio)-2,4,6-cyclohexantriol-trichlorid-dihydrat (12a)
Analyse:
$C_{12}H_{27}N_3O_3 \cdot 3HCl \cdot 2H_2O$

| | C | H | N | Cl |
|---|---|---|---|---|
| berechnet | 35.43% | 8.42% | 10.33% | 26.15% |
| gefunden | 35.28% | 8.04% | 10.23% | 26.40% |

$^1$H-NMR (90 MHz, $D_2O$):
5.0 ppm (m, 3H), 4.8 ppm (s, HDO), 3.6 ppm (m, 3H), 3.2 ppm (s, 18H).
$^{13}$C-NMR (62.9 MHz, $D_2O$, breitbandentkoppelt):
64.6 ppm, 61.8 ppm, 42.4 ppm.

all-cis-1,3,5-Tris(dimethylamino)-2,4,6-cyclohexantriol (11)
Smp: 118 – 119 °C
Analyse:

| $C_{12}H_{22}N_3O_3$ | C | H | N |
|---|---|---|---|
| berechnet | 55.15% | 10.41% | 16.08% |
| gefunden | 55.08% | 10.34% | 15.96% |

$^1$H-NMR (90 MHz, $CDCl_3$):
4.35 ppm (m, 3H), 3.8 ppm (m, 3H), 2.47 ppm (s, 18H), 1.73 ppm (m, 3H).
Schütteln mit $D_2O$ ergab folgende veränderte Signale:
4.7 ppm (s) neu, 4.35 ppm (m, 3H), kein Signal im Bereich 3.5 – 4.0 ppm.

$^1$H-NMR (90 MHz, $D_2O$):
4.80 ppm (s, HDO), 4.63 ppm (m, 3H), 2.45 ppm (s, 18H), 2.05 ppm (m, 3H).
$^{13}$C-NMR (62.9 MHz, $D_2O$, breitbandentkoppelt):
66.6 ppm, 66.3 ppm, 42.3 ppm.

Beispiel 7
all-cis-1,3,5-Tris-(dimethylammonio)-2-4-6-cyclohexantriol-sulfat (12b)

Das Sulfat (12b) wurde durch stöchiometrische Umsetzung des Amins (11) mit verdünnter Schwefelsäure erhalten. Es ist im Gegensatz zum Chlorid (12a) in siedendem Methanol praktisch unlöslich.

$^1$H-NMR (90 MHz, $D_2O$):
Identisch mit (12a)

Beispiel 8
Methylierung von all-cis-1,3,5-Triammonio-2,4,6-cyclohexan-triol-triformiat mit Formaldehyd und Ameisensäure
all-cis-1,3,5-Tris-(dimethylammonio)-2-4-6-cyclohexan-triol-trichlorid-dihydrat (12a)

13 g (38 mMol) Formiat (10c), 25 g (ca. 0.5 Mol) Ameisensäure (98 – 100%ig) und 25 g (0.33 Mol) wäßrige, 40%ige Formaldehydlösung wurden in 70 ml Wasser gelöst. Man erwärmte während 18 Stunden am Rückfluß auf 120 °C. Fünf Stunden nach dem Start gab man weitere 10 g (0.13 Mol) Formaldehydlösung zu. Anschließend engte man die klare Lösung am RV ein und nahm den Rückstand in 200 ml Etanolo auf. Nun wurde während einer Stunde trockener Chlorwasserstoff eingeleitet, dabei kristallisierte sich ein weißer Festkörper. Man ließ über Nacht bei 4 °C stehen un nutschte ab. Das Kristallisat wurde mit Alkohol und Ether gewaschen und am HV getrocknet.
Ausbeute: 4.5 g (11 mMol, 29%) (12a).

Die instrumentalanalytischen Eigenschaften dieses Produktes waren mit dem in Beispiel 6 beschriebenen Präparat identisch.

Beispiel 9
Quaternisierung von 1,3,5-Tris-(dimethylamino)-2-4-6-cyclohexantriol (11)

Quaternisierung mit Methyliodid
Die Quaternisierung wurde in Methanol, Acetonitril und Nitromethan bei Raumtemperatur oder am Rückfluß durchgeführt. Die triquartäre Verbindung wurde durch Umkristallisation in reiner Form erhalten.

$^1$H-NMR-Spektrum der monoquartären Verbindung (17):
(90 MHz, $D_2O$):
5.0 ppm (m, 2H), 4.8 ppm (s, HDO), 4.7 ppm (m, 1H), 3.5 ppm (s, 9H), 3.4 ppm (t, 1H), 2.5 ppm (s, 12H), 2.2 ppm (m, 2H).

$^1$H-NMR-Spektrum der diquartäten Verbindungen (18):
(90 MHz, $D_2O$):
5.45 ppm (m, 1H), 5.1 ppm (m, 2H), 4.8 ppm (s, HDO), 3.55 ppm (s, 18H), 3.45 (m, 2H), 2.6 ppm (s, 6H), 2.35 ppm (m, 1H).

Beispiel 10
all-cis-1,3,5-Tris-(trimetylammonio)-2-4-6-cyclohexantriol-triiodid (19a)

a) 200 mg (0.77 mMol) wasserfreies Triamin (11) wurden in 2 ml Nitromethan gelöst und anschließend mit 500 mg (3.5 mMol) Methyliodid versetzt. Dabei kristallisierte ein weißer Festkörper. Man rührte während 24 Stunden bei Raumtemperatur weiter und engte am RV zur Trockene ein. Das am HV getrocknete Produkt erwies sich als reines (19a).

b) 10 g (18 mMol) diquartäres (18) wurde in 80 ml Methanol und 20 ml Wasser suspendiert. Man gab 10 g (70 mMol) Methyliodid zu und kochte während 24 Stunden am Rückfluß. Anschließend wurde am RV zur Trockene eingeengt und dreimal wie folgt umkristallisiert: Man suspendierte den Festkörper in 40 ml Wasser. Nun gab man 0.1 M NaOH-Lösung zu, bis der pH-Wert etwa 8 bis 9 betrug. Man erwärmte, bis alles gelöst war und versetzte mit 100 ml Methanol. Bei 0 °C erhielt man farblose Kristalle. Die Verbindung kann auch aus siedendem Wasser umkristallisiert werden.

Analyse:

| $C_{15}H_{36}I_3N_3O_3 \cdot 0.5\ H_2O$ | C | H | N |
|---|---|---|---|
| berechnet | 25.88% | 5.36% | 6.04% |
| gefunden | 25.63% | 5.10% | 6.03% |

$^1$H-NMR (90 MHz, $D_2O$):
5.6 ppm (m, 3H), 4.8 ppm (s, HDO), 3.7 ppm (m, 3H), 3.65 ppm (s, 27H).

Beispiel 11
all-cis-1,3,5-Tris-(ammoniomethanphosphonsäure)-2,4,6-cyclohexantriol-trichlorid

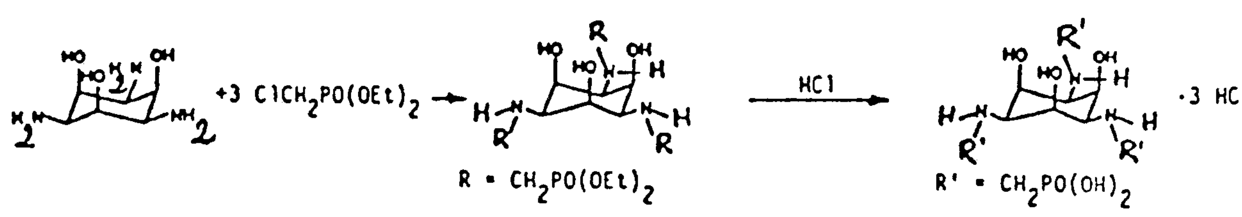


Eine Lösung von 177 mg (1 mMol) all-cis-1,3,5-Triamino-2,4,6-cyclohexantriol in 25 ml Wasser wurde mit 0.468 ml (3 mMol) Clormethanphosphonsäurediethylester versetzt und während 6 Tagen unter Stickstoff in der Dunkelheit am Rückfluß gekocht. Mit ca. 80% Ausbeute entstand all-cis-1,3,5-Tris-(ammoniomethanphosphonsäurediethylester)-2,4,6-cyclohexantriol-trichlorid.

$^1$H-NMR (250 MHz, $D_2O$, pD 4): 1.17 ppm (t), 3.19 ppm (m), 3.41 ppm (d), 3.88 ppm (m), 4.45 ppm (m)
$^{31}$P-NMR (100 MHz, $D_2O$, pD 4): 15.5 ppm
$^{13}$C-NMR (62.9 MHz, $D_2O$, pD 4): 15.9 ppm, 34.6 ppm, 50.4 ppm, 62.0 ppm, 65.2 ppm.
Durch mehrstündiges Kochen am Rückfluß in conc. HCl wird all-cis-Tris-(ammoniomethanphos-

phonsäure)-2,4,6-cyclohexantriol-trichlorid erhalten. $^{31}$P-NMR (101.3 MHz, $D_2O$, pD 4): 13.7 ppm
pK-Werte (ungefähr): 5.2, 6.0, 6.5, 7.0, 8.0, 9.5

Verhalten gegenüber Eisen:
Bei pH 7.5 hält der Ligand Eisen (III) in Lösung (maximal 2 Fe(III) pro Ligand). Eine Lösung mit FE(III): Ligand=0.9:1 ist klar im pH-Bereich von 2 bis 12. Zwischen pH 3 – 4 ist der Komplex relativ schwer löslich.

Beispiel 12
all-cis-1,3,5-Tris-(2'ammoniopropionsäure)-2,4,6-cyclohexantriol-trichlorid

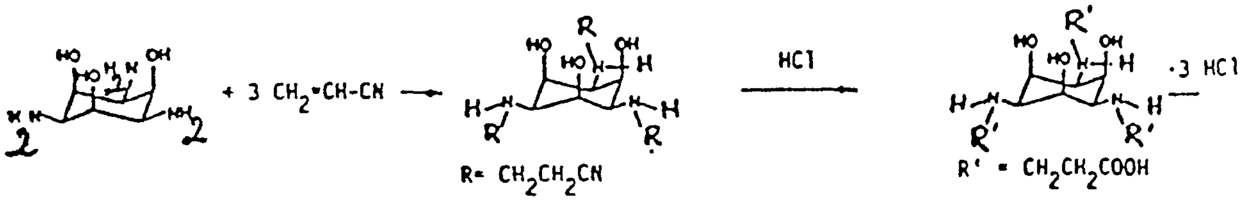


Eine Lösung von 0.177 g (1 mMol) all-cis-1,3,5-Triamino-2,4,6-cyclohexantriol in 40 ml Wasser wurde mit 3 ml einer 1 M Acrylonitrillösung (3 mMol) versetzt und während einer Woche unter $N_2$ im Dunklen stehengelassen. Durch Einengen am Rotavapor wurde das Zwischenprodukt al-cis-1,3,5-Tris-(2'aminopropionitril)-2,4,6-cyclohexantriol erhalten.
$^1$H-NMR (90 MHz, $D_2O$, pD 2): 4.54 ppm (m, 3H), 3.60 ppm (m,3H), 3.49 ppm (t,6H), 2.98 ppm (t,6H)
$^{13}$C-NMR (62.9 MHz, $D_2O$, pD 3): 14.9 ppm, 40.6 ppm. 57.0 ppm, 63.2 ppm, 117.4 ppm
pK-Werte (ungefähr): 3.0, 4.5, 6.3

Das Nitril wurde hydrolysiert durch Kochen am Rückfluß in conc. HCl während 3 Stunden. Durch Einengen am Rotapavor wurde das all-cis-1,3,5-Tris-(2'-ammoniopropionsäure)-2,4,6-cyclohexantriol-trichlorid erhalten.
$^{13}$C-NMR (62.9 MHz, $D_2O$, pD 4): 32.1 ppm, 42.3 ppm. 56.4 ppm, 63.1 ppm, 178.2 ppm
pK-Werte (ungefähr): 6.2, 8.2, 9.7

Verhalten gegenüber Eisen(III):
Bei pH 7.5 hält ein Ligandmolekül maximal 2 Fe(III) in Lösung.
Eine Lösung mit FE(III): Ligand=1:1 ist klar über die ganze pH-Skala. Zwischen pH 3 bis pH 6 ist der Komplex relativ schwer löslich.

Beispiel 13
all-cis-1,3,5-Tris-(ammonioessigsäure)-2,4,6-cyclohexantriol-trichlorid

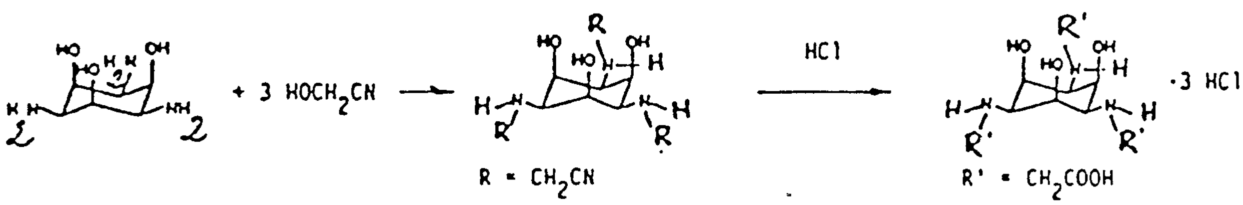


0.0975 g all-cis-1,3,5,-Triammonio-2,4,6-cyclohexantriol-sulfat (0.3 mMol) wurden in 60 ml Wasser gelöst mit 4.5 ml KOH 0.2 M (0.9 mMol) neutralisiert. Dazu gab man 0.0514 g frisch destilliertes Glykolsäurenitril (0.903 mMol) und liess die klare Lösung unter $N_2$ während 6 Tagen im Dunkeln stehen. Die leicht gelbliche Lösung wurde am Rotavapor zur Trockene eingeengt.

$^{13}$C-NMR (62.9 MHz, $D_2O$): 33.8 ppm, 57.7 ppm, 68.9 ppm, 119 ppm (breit)
pK-Werte (ungefähr): 6.6, nächste kleiner ca. 3.

Das Produkt wurde in 20 ml conc. HCl über Nacht am Rückfluß gekocht. Nach Einengen wurde bis zur Gewichtskonstanz getrocknet
pK-Werte (ungefähr): Drei kleiner als 3, 5.7, 7.8, 8.9.

Verhalten gegenüber Eisen(III):
Lösungen mit einem Ligand: Eisen-Verhältnis von 1:1 bleiben von pH 2 bis ca. 11 klar. Bei höherem pH-Wert fällt Eisenhydroxid aus.

Beispiel 14
all-cis-1,3,5-(N-methylammonioessigsäure)-2,4,6-cyclohexantriol-trichlorid

0.5 mMol all-cis-1,3,5-Tris-(aminoessigsäurenitril)-2,4,6-cyclohexantriol (Zwischenprodukt aus Beispiel 13) wurden in 20 ml Methanol gelöst. Dazu gab man 282 mg Methyliodid (2 mMol) und je 2 mMol $KHCO_3$ und $K_2CO_3$. Das Gemisch wurde über Nacht am Rückfluß gekocht. Nach Einengen zur Trockene, Ansäuern mit $HNO_3$ auf pH 1 ergab die Titration mit $AgNO_3$ einen Iodidgehalt von 80% des theoretischen Wertes für all-cis-1,3,5-Tri-(N-methylammonioacetonitril)-2,4,6-cyclohexantriol-triiodid. Die Methylierung war also noch nicht vollständig. Das Nitril wurde anschließend durch Kochen am Rückfluß in 20 ml conc. HCl zur Säure hydrolisiert.

Verhalten gegenüber $\theta$-FeO(OH):
Die Verbindung löst diesen Rost im Verhältnis 1:1 auf.

Beispiel 15
all-cis-1,3,5-(N-butylammonioessigsäure)-2,4,6-cyclohexantriol-trichlorid

Herstellung wie in Beispiel 14, aber mit 0.27 g n-Butylbromid statt mit Methyliodid. Die Titration mit $AgNO_3$ ergab annähernd den theoretischen Wert.

Verhalten gegenüber $\theta$-FeO(OH): identisch mit Beispiel 13 und 14.

Beispiel 16

Synthese von all-cis-1,3,5-Tris-(succinoylamino)-2,4,6-cyclohexantriol

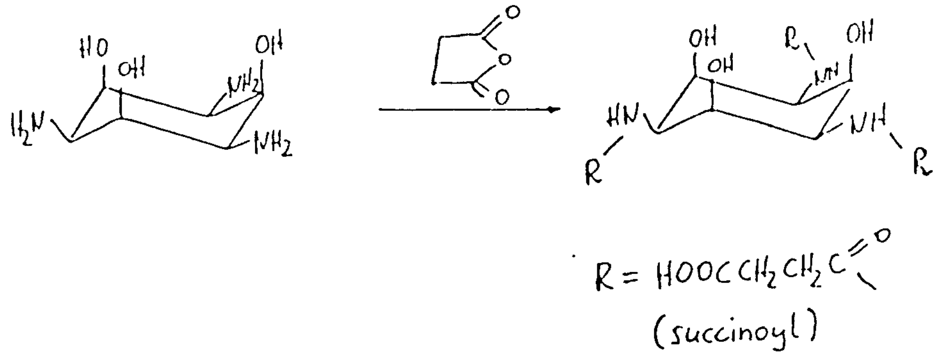


10 g (0.057 mol) all-cis Triamino-cyclohexantriol werden in 100 ml Wasser gelöst. Es werden 18,7 g (0.187 mol) Bernsteinsäureanhydrid in der minimalen Menge Wasser gelöst und langsam zugegeben. Das Reaktionsgemisch wird 30 Minuten gerührt und danach eingeengt auf etwa 20 ml. Der pH beträgt etwa 6,0. Man gibt 300 ml DMSO zu und zieht das restliche Wasser im Vakuum ab. Das DMSO verbleibt im Niederschlag, nachdem kein Wasser mehr vorhanden ist. Dieser Niederschlag wird abfiltriert und solange (ca. 16 h am Hochvakuum) getrocknet, bis die Substanz DMSO frei ist.

Ausbeute: 1. Fraktion: 7,3 g (0.015 mol) Produkt ≙ 28%. Aus der Mutterlauge kann durch Einengen und Abfiltration weiteres Produkt gewonnen werden.

Schmelzpunkt: > 300 °C

Löslichkeit: gut im Wasser, Wasser/MeOH, Wasser/EtOH, schlecht in Cyclohexan

UV: max. 212 nm

IR: CONH: 1650, 1555

Beispiel 17

Synthese von all-cis-1,3,5-Tris-(octanoylamino)-cyclohexantriol

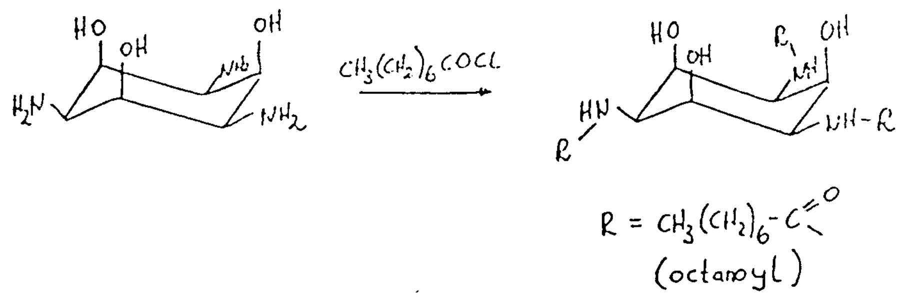


6 g (0.018 mol) all-cis Triamino-cyclohexantriol werden in einem Becherglas mit der minimalen Menge Wasser gelöst. Man gibt 20 ml (0.123 mol) Caprylsäurechlorid zu und rührt das Gemisch. Um eine gute Ausbeute zu erhalten, wird ein Überschuß von 1:2,3 gebraucht. Nun wird unter starkem Rühren 10 n Natronlauge zugegeben, bis ein weißer Niederschlag entsteht (z.T. NaCl, zum andern Teil Produkt). Durch Zugabe von Wasser löst man das Kochsalz, das Produkt aber bleibt als Niederschlag. Das Produkt ist wasserunlöslich. Der Niederschlag wir abfiltriert und erneut mit Wasser gewaschen. Zur besseren Reinigung löst man das Produkt in MeOH und fällt es erneut aus Wasser.

Ausbeute: 6.0 g Produkt entsprechend 0.011 mol ≙ 60%.

Schmelzpunkt: > 300 °C

Löslichkeit: gut in EtOH, $CHCl_3$, Tween, MeOH, unlöslich in $H_2O$

UV: max. 212 nm

IR: CONH: 1630, 1530

Pharmakologische Wirkung

all-cis-1,3,5-Triamino-2,4,6-cyclohexantriol (10), all-cis-1,3,5-Tris-(dimethylamino)-2,4,6-cyclohexantriol-Hydrochlorid (12a), all-cis-1,3,5-Tris-(trimethylammonio)-2,4,6-cyclohexantriol (19c) und all-cis-1,3,5-Tris-(succinoylamino)-2,4,6-cyclohexantriol weisen im orientierenden $LD_{50}$-Toxizitätstest an der weißen Maus nach i.v.-Gabe folgende Werte im mg/kg Körpergewicht auf: 158, 440, 235 und > 1000. Die Eisenelimination im Vergleich zu Desferal nach i.v.-Gabe aus eisenüberladenen Ratten in den Harn resp. Faeces kann folgender Tabelle entnommen werden. Dabei wurden die Ausscheidungsraten über 72 Stunden post applikationem gemessen.

| Substanz | Harn | Faeces |
|---|---|---|
| Desferal | 1 | 1 |
| 10 | 0,1 | 0,2 |
| 12a | 1 | 0,5 |
| 19c | 0,2 | 0,5 |

**Patentansprüche**

1. all-cis-1,3,5-Triamino-2,4,6-trihydroxycyclohexan-Derivate der allgemeine Formel I

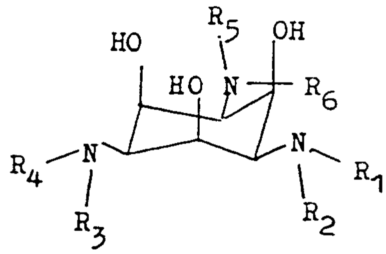

I

worin die Symbole $R_1$, $R_2$, $R_3$, $R_4$, $R_5$ und $R_6$ gleich oder verschieden sind und für Wasserstoffatome, Alkylgruppen oder -CO-Alkylgruppen stehen, wobei das Alkyl in den Alkyl- oder -CO-Alkylgruppen 1 bis 18 Kohlenstoffatome aufweist und wobei die Alkyl- und -CO-Alkylgruppen jeweils unabhängig voneinander eine oder mehrere, gleiche oder verschiedene funktionelle Gruppen enthalten können und wobei zumindest einer der Reste $R_1$ bis $R_6$ eine der vorstehenden unsubstituierten oder substituierten Alkylgruppen oder -CO-Alkylgruppen ist, sowie deren Salze mit anorganischen oder organischen pharmakologisch üblichen Säuren und deren quaternäre Ammoniumsalze der allgemeinen Formel II, IIa oder IIb

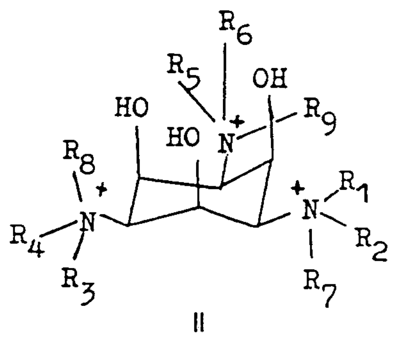

II

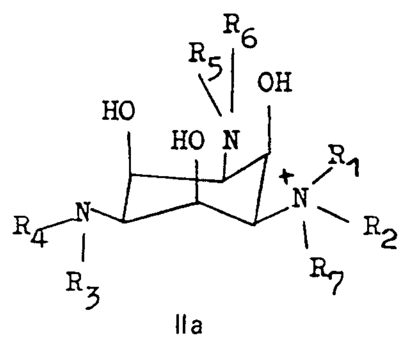

IIa

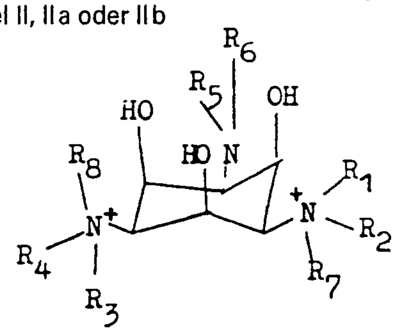

IIb

mit pharmazeutisch brauchbaren Anionen, worin $R_1$ bis $R_6$ sowie $R_7$, $R_8$ und $R_9$ unabhängig voneinander die vorstehend definierten unsubstituierten oder substituierten Alkylgruppen oder -CO-Alkylgruppen bedeuten, mit Ausnahme der Verbindung, worin $R_1 = R_3 = R_5 =$ -$COCH_3$ und $R_2 = R_4 = R_6 =$ -H.

2. Derivate nach Anspruch 1, dadurch gekennzeichnet, daß das Alkyl 1 bis 12 Kohlenstoffatome aufweist.

3. Derivate nach Anspruch 1, dadurch gekennzeichnet, daß das Alkyl 1 bis 6 Kohlenstoffatome aufweist.

4. Derivate nach Anspruch 1, dadurch gekennzeichnet, daß das Alkyl 1 bis 4 Kohlenstoffatome aufweist und bevorzugt Methyl, Ethyl, n-Propyl, Isopropyl, n-Butyl und/oder tert.-Butyl ist.

5. Derivate nach einem oder mehreren der Ansprüche 1 bis 4, dadurch gekennzeichnet, daß von den Symbolen $R_1$ bis $R_9$ an jedem Stickstoffatom höchstens jeweils ein Symbol einen sekundären oder einen tertiären Alkylsubstituenten oder eine -CO-Alkylgruppe darstellt.

6. Derivate nach Anspruch 1, dadurch gekennzeichnet, daß man an einem oder an mehreren der Stickstoffatome jeweils ein Symbol einen sekundären oder einen tertiären Alkylsubstituenten oder eine -CO-Alkylgruppe darstellt und daß die anderen Symbole die gleiche Bedeutung wie gemäß Anspruch 1 haben.

7. Derivate nach Anspruch 6, dadurch gekennzeichnet, daß die anderen Symbole für Wasserstoff oder -$CH_3$ stehen.

8. Derivate nach Anspruch 1, dadurch gekennzeichnet, daß sämtliche der Reste $R_1$ bis $R_6$ bzw. $R_1$ bis $R_9$ gleich und über ein primäres C-Atom gebundene Alkylgruppen sind.

9. Derivate nach Anspruch 1, dadurch gekennzeichnet, daß sämtliche der Reste $R_1$ bis $R_6$ bzw. $R_1$ bis $R_9$ Methyl- oder -$COCH_3$-Gruppen sind.

10. Derivate nach einem oder mehreren der Ansprüche 1 bis 9, dadurch gekennzeichnet, daß die Alkylgruppen oder -CO-Alkylgruppen eine oder mehrere funktionelle Gruppen enthalten, die an Metallkationen, insbesondere an Eisen (III) koordinieren können.

11. Derivate nach einem oder mehreren der Ansprüche 1 bis 9, dadurch gekennzeichnet, daß die funktionellen Gruppen an den Alkylgruppen oder -CO-Alkylgruppen -OH, -COOH oder Salze davon, -$CONH_2$, -CON(OH)R, wobei R für eine Alkylgruppe mit 1 bis 6, insbesondere mit 1 bis 4 Kohlenstoffatomen steht, -$OPO_3H_2$ oder deren Salze, -$PO_3H_2$ oder deren Salze, -SR (worin R die obige Bedeutung hat), sowie die Ester der vorstehenden Säuren, -CN,

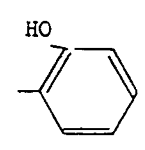

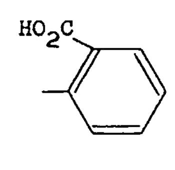

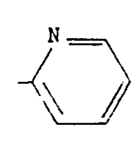

und/oder

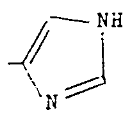

und/oder deren Salze sind.

12. Derivate nach einem oder mehreren der Ansprüche 1 bis 11, dadurch gekennzeichnet, daß eines, mehrere oder alle der Symbole $R_1$ bis $R_9$ unabhängig voneinander folgende Bedeutungen haben:

a) -H

b) -Alkylm mit 1 – 12 C-Atomen im Alkyl oder -CO-Alkyl mit 1–12 C-Atomen im Alkyl

c) $-(CH_2)_nOH$

d) $-(CH_2)_nCO_2^-$

e) $-(CH_2)_nCONH_2$

f) $-(CH_2)_nCON(OH)R$

R=Alkyl mit
$C_{1-12}$,
bevorzugt $C_{1-4}$

g) $-(CH_2)_nOPO^{2-}{}_3$

h) $-(CH_2)_nPO^{2-}{}_3$

i) $-(CH_2)_nSR$

R=Alkyl mit
$C_{1-12}$,
bevorzugt $C_{1-4}$

j) 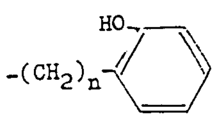

k) 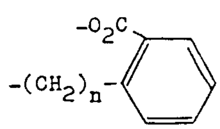

l) 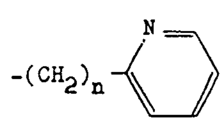

m) 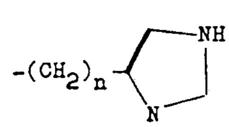

wobei n 1, 2 oder 3 ist sowie deren Salze.

13. Derivate nach einem oder mehreren der Ansprüche 1 bis 11, dadurch gekennzeichnet, daß eines, mehrere oder alle der Symbole $R_1$ bis $R_9$ unabhängig voneinander folgende Bedeutungen haben:

a) $-CO-(CH_2)_nOH$

b) $-CO-(CH_2)_nCO\ _2$

c) $-CO-(CH_2)_nCONH_2$

d) $-CO-(CH_2)_nCON(OH)R$

R=Alkyl mit
$C_{1-12}$,
bevorzugt $C_{1-4}$

e) $-CO-(CH_2)_nOPO^2\ _3$

f) $-CO-(CH_2)_nPO^2\ _3$

g) $-CO-(CH_2)_nSR$

R=Alkyl mit
$C_{1-12}$,
bevorzugt $C_{1-4}$

h) 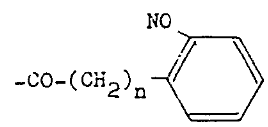

i) 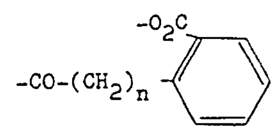

j) 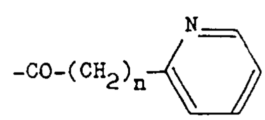

k) 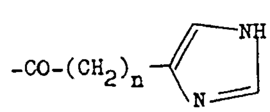

wobei n 1, 2 oder 3 ist sowie Salze.

14. all-cis-1,3,5-Triamino-2,4,6-cyclohexantriol-Derivate der allgemeinen Formel

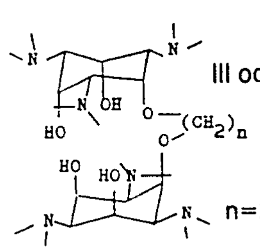 III oder 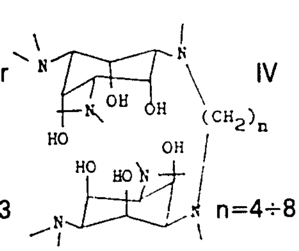 IV

n=2,3 n=4÷8

worin die Substituenten an den Stickstoffatomen unabhängig voneinander H oder $CH_3$ sind.

15. Derivate nach Anspruch 14, worin alle Substituenten an den Stickstoffatomen $CH_3$-Gruppen sind.

16. Pharmazeutische Präparate bzw. Arzneimittel, enthaltend eine oder mehrere der Verbindungen nach einem der Ansprüche 1 bis 15 und/oder all-cis-1,3,5-Triamino-2,4,6-cyclohexantriol, gegebenenfalls in der Form eines pharmakologisch brauchbaren Salzes, zusammen mit üblichen Hilfs- und Trägerstoffen.

17. Verwendung der Verbindungen nach einem der Ansprüche 1 bis 15 und/oder von all-cis-1,3,5-Triamino-2,4,6-cyclohexantriol, gegebenenfalls in der Form pharmakologisch brauchbarer Salze, zur Herstellung von Arzneimitteln für die therapeutische Anwendung bei Eisenüberladungen im tierischen und menschlichen Körper.

18. Verwendung der Verbindungen nach einem der Ansprüche 1 bis 15 und/oder von all-cis-1,3,5-Triamino-2,4,6-cyclohexantriol als Liganden für Metallionen, insbesondere als Ionen-Carrier in ionenselektiven Elektroden.

19. Verwendung der Verbindungen nach einem der Ansprüche 1 bis 15 und/oder von all-cis-1,3,5-Triamino-2,4,6-cyclohexantriol zur Entfernung unerwünschter Eisenablagerungen in industriellen Anlagen oder Kernkraftwerken.

20. Verwendung der Verbindungen nach einem der Ansprüche 1 bis 15 und/oder von all-cis-1,3,5-Triamino-2,4,6-cyclohexantriol als Liganden für Metallionen, insbesondere für therapeutische oder diagnostische Anwendungen.

21. Verwendung nach Anspruch 19 bis 20 für Erdalkaliionen, insbesondere Magnesium.

22. Verwendung nach Anspruch 20 für Ionen von seltenen Erden, insbesondere Gadolinium.

23. Verwendung nach Anspruch 20 für Ionen von Aluminium, Gallium, Indium oder Technetium.

24. Verfahren zur Herstellung von all-cis-1,3,5-Triamino-2,4,6-cyclohexantriol und seinen alkylierten Derivaten gemäß einem der Ansprüche 1 bis 15, dadurch gekennzeichnet, daß man

a) Trinitrophloroglucin oder sein Monoalkalimetallsalz katalytisch hydriert,

b) gegebenenfalls das erhaltene Produkt in Form des freien Amins oder seines Salzes alkyliert und

c) gegebenenfalls das erhaltene Produkt, insbesondere das alkylierte Produkt, zu den entsprechenden mono- di- oder tri-quartären Verbindungen alkyliert.

25. Verfahren nach Anspruch 24, dadurch gekennzeichnet, daß die katalytische Hydrierung a) von Trinitrophloroglucin in Gegenwart von Platin mit Wasserstoff durchgeführt wird.

26. Verfahren nach Anspruch 24 oder 25, dadurch gekennzeichnet, daß die Alkylierung b) mit einem Alkylhalogenid oder einem Olefin oder Alkohol in einer Additions- bzw. Kondensationsreaktion durchgeführt wird.

27. Verfahren nach einem der Ansprüche 24 bis 26, dadurch gekennzeichnet, daß zur Herstellung der Diamine der allgemeinen Formel IV nach Anspruch 14 die Alkylierung mit einem difunktionellen Alkylierungsmittel, insbesondere einem Alkylenhalogenid, durchgeführt wird, wobei gegebenfalls zwei Moleküle des als Ausgangsmaterial verwendeten all-cis-1,3,5-Triamino-2,4,6-cyclohexantriols in Form eines Komplexes mit Eisen (III) oder Chrom (III) vorliegen und daß zur Herstellung der Diether der allgemeinen Formel III nach Anspruch 14 die Alkylierung ausgehend von einem quaternisierten Derivat der allgemeinen Formel II durchgeführt wird.

28. Verfahren nach einem der Ansprüche 24 bis 27, dadurch gekennzeichnet, daß die katalytische Hydrierung unter intensivem, eine Turbulenz erzeugendem Bewegen durchgeführt wird.

29. Verfahren nach einem der Ansprüche 24, 25, 27 und 28, dadurch gekennzeichnet, daß die Alkylierung der Stufe b) reduktiv mit Aldehyden oder Ketonen durchgeführt wird.

30. Verfahren nach Anspruch 29, dadurch gekennzeichnet, daß die reduktive Alkylierung entweder mit Ameisensäure oder in wäßriger Lösung mit $H_2/Pt$ durchgeführt wird.

31. Verfahren nach einem der Ansprüche 24 bis 30, dadurch gekennzeichnet, daß die Alkylierung der Stufe c) mit einem Alkylhalogenid durchgeführt wird, insbesondere mit Methyljodid.

32. Verfahren nach einem der Ansprüche 24 bis 31, dadurch gekennzeichnet, daß Trinitrophloroglucin eingesetzt wird, das erhalten wurde durch

a) Umsetzung von Phloroglucin mit Acetanhydrid und

b) Nitrieren des erhaltenen Triacetats mit rauchender Salpetersäure.

**Claims**

1. all-cis-1,3,5-triamino-2,4,6-trihydroxycyclohexane derivatives corresponding to general formula I

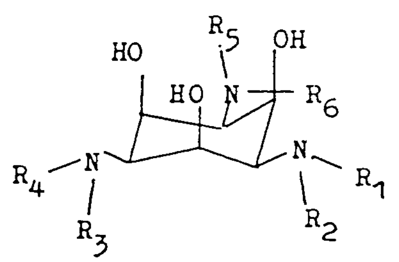

I

in which the symbols $R_1$, $R_2$, $R_3$, $R_4$, $R_5$ and $R_6$ are the same or different and represent hydrogen atoms, alkyl groups or -CO-alkyl groups, the alkyl in the alkyl and -CO-alkyl groups containing from 1 to 18 carbon atoms and the alkyl and -CO-alkyl groups independently of one another optionally containing one or more, like or different functional groups and at least one of the symbols $R_1$ to $R_6$ representing one of the above-mentioned unsubstituted or substituted alkyl groups or -CO-alkyl groups, and salts thereof with inorganic or organic, pharmacologically typical acids and quaternary ammonium salts thereof corresponding to general formula II, IIa or IIb

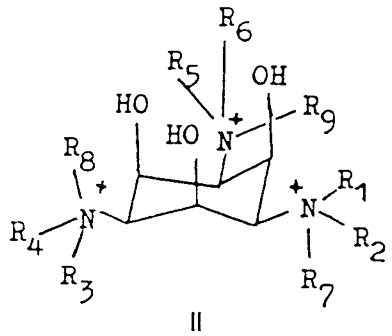

II

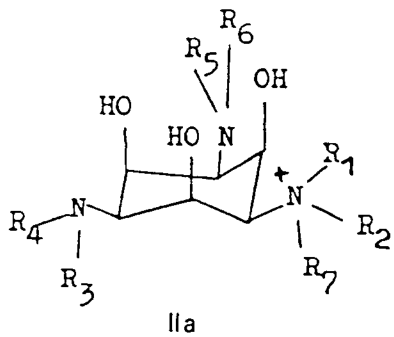

IIa

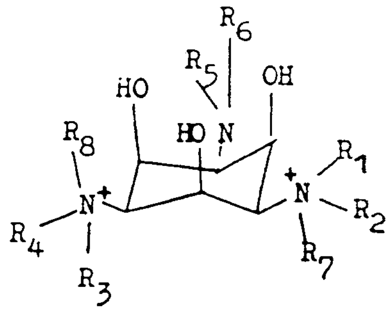

IIb

with pharmaceutically useable anions, in which $R_1$ to $R_6$ and $R_7$, $R_8$ and $R_9$ independently of one another represent the unsubstituted or substituted alkyl groups defined above or -CO-alkyl groups, except for the compound in which $R_1=R_3=R_5=-COCH_3$ and $R_2=R_4=R_6=$ -H.

2. Derivatives as claimed in claim 1, characterized in that the alkyl contains 1 to 12 carbon atoms.

3. Derivatives as claimed in claim 1, characterized in that the alkyl contains 1 to 6 carbon atoms.

4. Derivatives as claimed in claim 1, characterized in that the alkyl contains 1 to 4 carbon atoms and is preferably methyl, ethyln n-propyl, isopropyl, n-butyl and/or tert. butyl.

5. Derivatives as claimed in one or more of claims 1 to 4, characterized in that, of the symbols $R_1$ to $R_9$ at each nitrogen atom, at most one symbol represents a secondary or tertiary alkyl substituent or a -CO- alkyl group.

6. Derivatives as claimed in claim 1, characterized in that, at one or more of the nitrogen atoms, one symbol represents a secondary or tertiary alkyl substituent or a -CO- alkyl group and in that the other symbols have the same meaning as in claim 1.

7. Derivatives as claimed in claim 6, characterized in that the other symbols represent hydrogen or $-CH_3$.

8. Derivatives as claimed in claim 1, characterized in that all the substituents $R_1$ to $R_6$ or $R_1$ to $R_9$ are the same and are alkyl groups attached by a primary C atom.

9. Derivatives as claimed in claim 1, characterized in that all the substituents $R_1$ to $R_6$ or $R_1$ to $R_9$ are methyl or $-COCH_3$- groups.

10. Derivatives as claimed in one or more of claims 1 to 9, characterized in that the alkyl groups or -CO-alkyl groups contain one or more functional groups which may coordinate at metal cations, particularly at iron (III).

11. Derivatives as claimed in one or more of claims 1 to 9, characterized in that the functional groups at the alkyl groups ore -CO- alkyl groups are -OH, -COOH or salts thereof, $-CONH_2$, -CON(OH)R, where R is an alkyl group containing 1 to 6 and more especially 1 to 4 carbon atoms, $-OPO_3H_2$ or salts thereof, $-PO_3H_2$ or salts thereof, -SR (where R is as definded above) and also esters of the above acids, -CN,

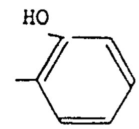

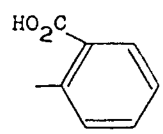

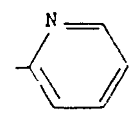

and/or

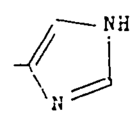

and/or salts thereof.

12. Derivatives as claimed in one or more of claims 1 to 11, characterized in that one, several or all of the symbols $R_1$ bis $R_9$ independently of one another have the following meanings:

a) -H

b) -alkyl with 1 – 12 C atoms
or -CO-alkyl with 1 – 12 C atoms in the alkyl

c) $-(CH_2)_nOH$

d) $-(CH_2)_nCO_2^-$

e) $-(CH_2)_nCONH_2$

f) $-(CH_2)_nCON(OH)R$
$R=C_{1-12}$,
preferably $C_{1-4}$ alkyl

g) $-(CH_2)_nOPO^{2}{}_3$

h) $-(CH_2)_nPO^{2}{}_3$

i) $-(CH_2)_nSR$

$R=C_{1-12}$,
preferably $C_{1-4}$ alkyl

j) 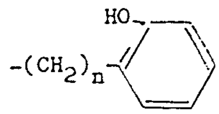

k) 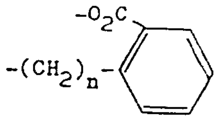

l) 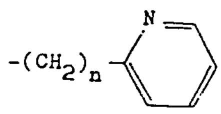

m) 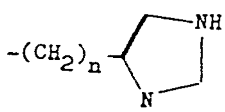

n=1, 2 or 3, and salts thereof.

13. Derivatives as claimed in one or more of claims 1 to 11, characterized in that one, several or all of the symbols $R_1$ bis $R_9$ independently of one another have the following meanings:

a) $-CO-(CH_2)_nOH$

b) $-CO-(CH_2)_nCO^-_2$

c) $-CO-(CH_2)_nCONH_2$

d) $-CO-(CH_2)_nCON(OH)R$

$R=C_{1-12}$,
preferably $C_{1-4}$ alkyl

e) $-CO-(CH_2)_nOPO^{2-}_3$

f) $-CO-(CH_2)_nPO^{2-}_3$

g) $-CO-(CH_2)_nSR$

$R=C_{1-12}$,
preferably $C_{1-4}$ alkyl

h) 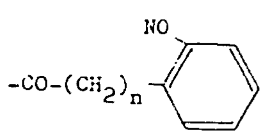

i) 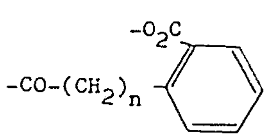

j) 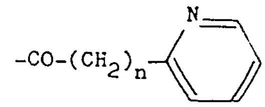

k) 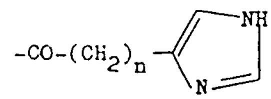

n 1, 2 or 3, and salts thereof.

14. all-cis-1,3,5-triamino-2,4,6-cyclohexantriol derivatives corresponding to the following general formula

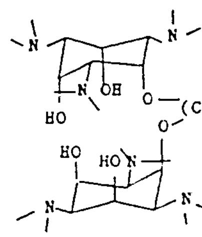 III or 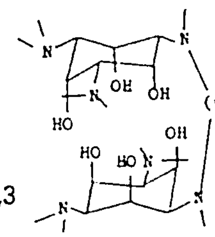 IV

n=2,3 n=4÷8

in which the substituents at the nitrogen atoms independently of one another are H oder $CH_3$.

15. Derivatives as claimed in claim 14, in which all the substituents at the nitrogen atoms are $CH_3$ groups.

16. Pharmaceutical preparations or medicaments containing one or more of the compounds claimed in any of claims 1 to 15 and/or all-cis-1,3,5-triamino-2,4,6-cyclohexanetriol, optionally in the form of a pharmaceutically useable salt, together with standard auxiliaries and excipients.

17. The use of the compounds claimed in any of claims 1 to 15 and/or of all-cis-1,3,5-triamino-2,4,6-cyclohexanetriol, optionally in the form of pharmacologically useable salts, for the preparation of medicaments for therapeutic application in cases of iron overloads in the animal or human body.

18. The use of compounds as claimed in any of claims 1 to 15 and of all-cis-1,3,5-triamino-2,4,6-cyclohexanetriol as ligands for metal ions and particularly as ion carriers in ion-selective electrodes.

19. The use of the compounds claimed in any of claims 1 to 15 and/or of all-cis-1,3,5-triamino-2,4,6-cyclohexanetriol for the removal of unwanted iron deposits in industrial plants or nuclear power stations.

20. The use of the compounds claimed in one or more of claims 1 to 15 and/or of all-cis-1,3,5-triamino-2,4,6-cyclohexanetriol as ligands for metal ions, particularly for therapeutic or diagnostic applications.

21. The use claimed in claims 19 and 20 for alkaline earth ions, particularly magnesium.

22. The use claimed in claim 20 for ions of rare earths, particularly gadolinium.

23. The use claimed in claim 20 for ions of aluminium, gallium, indium or technetium.

24. A process for the preparation of all-cis-1,3,5-triamino-2,4,6-cyclohexanetriol and its alkylated derivatives according to any of claims 1 to 15, characterized in that
a) trinitrophloroglucinol or its monoalkali metal salt is catalytically hydrogenated,
b) the product obtained is optionally alkylated or acylated in the form of the free amine or its salts and
c) the product obtained, particularly the alkylated product, is optionally alkylated to the corresponding mono- di- or tri-quaternary compounds.

25. A process as claimed in claim 24, characterized in that the catalytic hydrogenation a) of trinitrophloroglucinol is carried out with hydrogen in the presence of platinum.

26. A process as claimed in claims 24 or 25, characterized in that the alkylation b) is carried out with an alkyl halide or an olefin or alcohol in an addition or condensation reaction.

27. A process as claimed in any of claims 24 to 26, characterized in that, to prepare the diamines of general formula IV according to claim 14, the alkylation is carried out with a difunctional alkylating agent, particularly an alkylene halide, two molecules of the all-cis-1,3,5-triamino-2,4,6-cyclohexanetriol used as starting material optionally being present in the form of a complex with iron-(III) or chromium- (III), and in that, to prepare the diether of general formula II according to claim 14, the alkylation is carried out using a quaternized derivative of general formula II as starting material.

28. A process as claimed in any of claims 24 to 27, characterized in that the catalytic hydrogenation is carried out with intensive turbulence-generating agitation.

29. A process as claimed in any of claims 24, 25, 27 and 28, characterized in that the alkylation of step b) is carried out reductively with aldehydes or ketones.

30. A process as claimed in claim 29, characterized in that the reductive alkylation is carried out either with formic acid or in aqueous solution with $H_2/Pt$.

31. A process as claimed in any of claims 24 to 30, characterized in that the alkylation of step c) is carried out with an alkyl halide, more especially methyl iodide.

32. A process as claimed in any of claims 24 to 31, characterized in that trinitrophloroglucinol obtained by
a) reaction of phloroglucinol with acetanhydride and
b) nitration of the triacetate obtained with fuming nitric acid is used.

## Revendications

1. Dérivés entièrement cis du -1,3,5-triamino-2,4,6-trihydroxycyclohexane répondant à la formule générale I

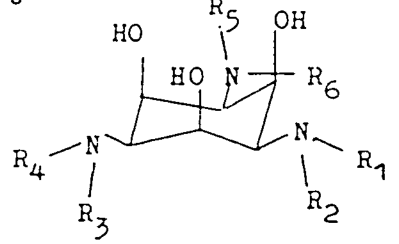

I

dans laquelle les symboles $R_1$, $R_2$, $R_3$, $R_4$, $R_5$ et $R_6$, ayant des significations identiques ou différentes, représentent' des atomes d'hydrogène, des groupes alkyle ou -CO-alkyle, ces groupes alkyle et les parties alkyle des groupes -CO-alkyle contenant 1 à 18 atomes de carbone et les groupes alkyle et -CO-alkyle pouvant contenir chacun, indépendamment les uns des autres, un ou plusieurs groupes fonctionnels identiques ou différents, l'un au moins des symboles $R_1$ à $R_6$ représentant un de ces groupes alkyle ou -CO-alkyle substitués ou non, leurs sels d'acides minéraux ou organiques pharmacologiques usuels et leurs sels d'ammonium quaternaires de formule générale II, IIa ou IIb

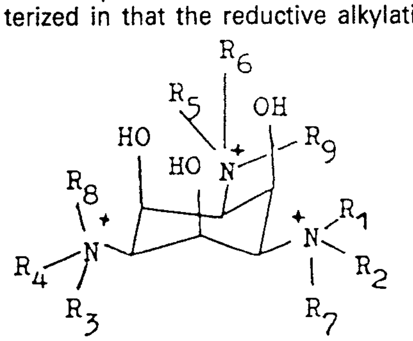

II

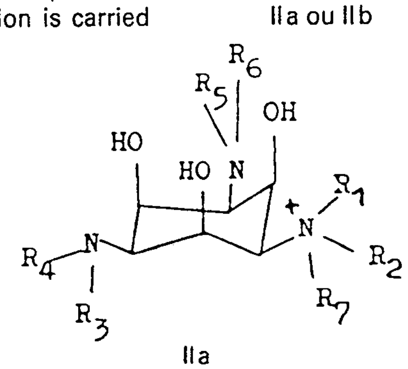

IIa

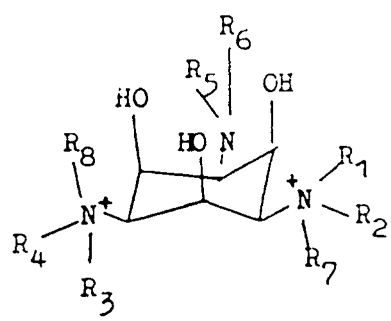

IIb

ayant des anions acceptables pour l'usage pharmaceutique, les symboles $R_1$ à $R_6$ ainsi que $R_7$, $R_8$ et $R_9$ représentant chacun, independamment les uns des autres, les groupes alkyle ou -CO-alkyle substitués ou non tels que définis ci-dessus, à l'exception du composé pour lequel $R_1=R_3=R_5=$ $-COCH_3$ and $R_2=R_4=R_6=$ -H.

2. Dérivés selon la revendication 1, caractérisés en ce que les groupes alkyle contiennent 1 à 12 atomes de carbone.

3. Dérivés selon la revendication 1, caractérisés en ce que les groupes alkyle contiennent 1 à 6 atomes de carbone.

4. Dérivés selon la revendication 1, caractérisés en ce que les groupes alkyle contiennent 1 à 4 atomes de carbone et sont de préférence des groupes methyle, ethyle, n-propyle, isopropyle, n-butyle et/ou tert-butyle.

5. Dérivés selon une ou plusieurs des revendications 1 à 4, caractérisés en ce que, sur chacun des

atomes d'azote, un seul au maximum des symboles $R_1$ à $R_9$ représente un substituant alkyle secondaire ou tertiaire ou un groupe -CO-alkyle.

6. Dérivés selon la revendication 1, caractérisés en ce que, sur un ou plusieurs des atomes d'azote, un symbole représente à chaque fois un substituant alkyle secondaire ou tertiaire ou un groupe -CO-alkyle et les autres symboles ont les significations indiquées dans la revendication 1.

7. Dérivés selon la revendication 6, caractérisés en ce que les autres symboles représentent l'hydrogène ou des groupes $-CH_3$.

8. Dérivés selon la revendication 1, caractérisés en ce que tous les symboles $R_1$ à $R_6$ ou $R_1$ à $R_9$ ont des significations identiques et représentent des groupes alkyle reliés par un atome de carbone primaire.

9. Dérivés selon la revendication 1, caractérisé en ce que tous les symboles $R_1$ à $R_6$ ou $R_1$ à $R_9$ représentent des groupes méthyle ou $-COCH_3$.

10. Dérivés selon une ou plusieurs des revendications 1 à 9, caractérisés en ce que les groupes alkyle ou -CO-alkyle contiennent un ou plusieurs groupes fonctionnels qui peuvent être reliés par des liaisons de coordination à des cations métalliques, en particulier à des cations fer-(III).

11. Dérivés selon une ou plusieurs des revendications 1 à 9, caractérisés en ce que les groupes fonctionnels contenus dans les groupes alkyle ou -CO-alkyle sont des groupes -OH, -COOH ou ses sels, $-CONH_2$, -CON(OH)R dans lequel R représente un groupe alkyle en C1 – C6, plus spécialement en C1 – C4, $-OPO_3H_2$ ou ses sels, $-PO_3H_2$ ou ses sels, -SR (dans lequel R a les significations indiquées ci-dessus) ainsi que les esters des acides ci-dessus, CN,

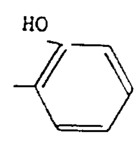

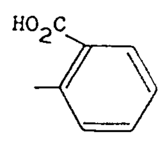

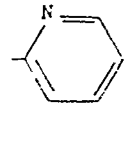

et/ou

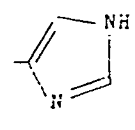

et/ou leurs seuls.

12. Dérivés selon une ou plusieurs des revendications 1 à 11, caractérisés en ce qu'un, plusieurs ou tous les symboles $R_1$ à $R_9$ ont, indépendamment les uns des autres, les significations suivantes:

a) -H

b) -alkyle en $C_1 - C_{12}$ ou -CO-alkyle contenant 1 à 12 atomes de C dans la partie alkyle

c) $-(CH_2)_nOH$

d) $-(CH_2)_nCO_2^-$

e) $-(CH_2)_nCONH_2$

f) $-(CH_2)_nCON(OH)R$

R=alkyle en $C_1 - C_{12}$,
de préférence $C_{1-4}$

g)$-(CH_2)_nOPO^{2-}_3$

h) $-(CH_2)_nPO^{2-}_3$

i) $-(CH_2)_nSR$

R=alkyle en $C_1 - C_{12}$,
de préférence $C_{1-4}$

j) 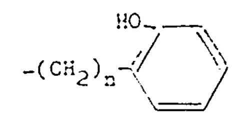

k) 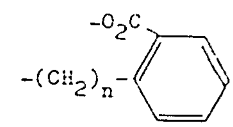

l) 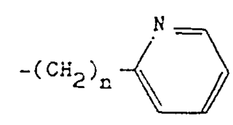

m) 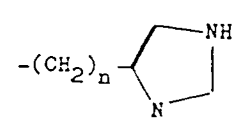

dans lesquelles n est égal à 1, 2 ou 3, et leurs sels.

13. Dérivés selon une ou plusieurs des revendications 1 à 11, caractérisés en ce qu'un, plusieurs ou tous les symboles $R_1$ à $R_9$ ont, indépendamment les uns des autres, les significations suivantes:

a) $-CO-(CH_2)_nOH$

b) $-CO-(CH_2)_nCO_2$

c) $-CO-(CH_2)_nCONH_2$

d) $-CO-(CH_2)_nCON(OH)R$

R=alkyle en $C_1 - C_{12}$, de préférence $C_{1-4}$

e) $-CO-(CH_2)_nOPO^{2}{}_{3}$

f) $-CO-(CH_2)_nPO^{2}{}_{3}$

g) $-CO-(CH_2)_nSR$

R=alkyle en $C_1 - C_{12}$, de préférence $C_{1-4}$

h) 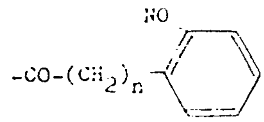

i) 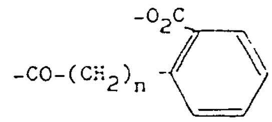

j) 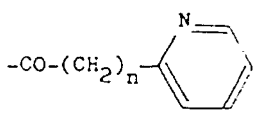

k) 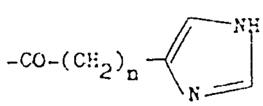

dans lesquelles n est égal à 1, 2 ou 3, et leurs sels.

14. Dérivés entièrement cis du 1,3,5-triamino-2,4,6-cyclohexane-triol de formule générale

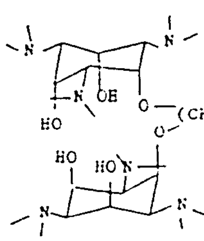 III ou 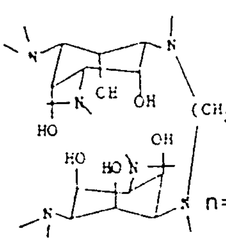 IV

n=2,3 n=4÷8

dans lesquelles les substituants des atomes d'azote, indépendamment les uns des autres, sont H ou $CH_3$.

15. Dérivés selon la revendication 14, dans lesquels tous les substituants des atomes d'azote sont des groupes $CH_3$.

16. Compositions pharmaceutiques ou médicaments contenant un ou plusieurs des composés selon l'une des revendications 1 à 15 et/ou du tout-cis-1,3,5-triamino-2,4,6-cyclohexane-triol, éventuellement à l'état de sel acceptable pour l'usage pharmaceutique, avec des produits auxiliaires et véhicules usuels.

17. Utilisation des composés selon l'une des revendications 1 à 15 et/ou du tout-cis-1,3,5-triamino-2,4,6-cyclohexane-triol, éventuellement à l'état de sels acceptables pour l'usage pharmaceutique, pour la préparation de médicaments pour l'utilisation thérapeutique dans les cas de surcharges en fer de l'organisme humain ou animal.

18. Utilisation des composés selon l'une des revendications 1 à 15, et/ou du tout-cis-1,3,5-triamino-2,4,6-cyclohexane-triol en tant que ligands pour des ions métalliques, en particulier en tant que véhicules d'ion dans des électrodes sélectives à l'égard des ions.

19. Utilisation des composés selon l'une des revendications 1 à 15 et/ou du tout-cis-1,3,5-triamino-2,4,6-cyclohexane-triol pour l'élimination des dépôts indésirables de fer dans des installations industrielles ou des usines nucléaires.

20. Utilisation des composés selon l'une des revendications 1 à 15 et/ou du tout-cis-1,3,5-triamino-2,4,6-cyclohexane-triol en tant que ligands pour des ions métalliques, en particulier dans des applications thérapeutiques ou diagnostiques.

21. Utilisation selon la revendication 19 ou 20 pour des ions alcalino-terreux, en particulier le magnésium.

22. Utilisation selon la revendication 20 pour des ions de terres rares, en particulier le gadolinium.

23. Utilisation selon la revendication 20, pour les ions d'aluminium, de gallium, d'indium ou de téchnetium.

24. Procédé de préparation du tout-cis-1,3,5-triamino-2,4,6-cyclohexane-triol et de ses dérivés alkylés selon l'une des revendications 1 à 15, caractérisé en ce que

a) on soumet le trinitrophloroglucinol ou l'un de ses sels mono-alcalins à hydrogénation catalytique,

b) on alkyle ou on acyle éventuellement le produit obtenu à l'état de l'amine libre ou son sel et

c) on alkyle éventuellement le produit obtenu, en particulier le produit alkylé, en les dérivés correspondants mono- di- ou tri-quaternaires.

25. Procédé selon la revendication 24, caractérisé en ce que l'hydrogénation catalytique a) du trinitrophloroglucinol est effectuée à l'aide d'hydrogène en présence de platine.

26. Procédé selon la revendication 24 ou 25, caractérisé en ce que l'alkylation b) est effectuée à l'aide d'un halogénure d'alkyle ou d'une oléfine ou d'un alcool dans une réaction respective d'addition ou de condensation.

27. Procédé selon l'une des revendications 24 à 26, caractérisé en ce que, pour préparer les diamines de formule générale IV selon la revendication 14, on effectue l'alkylation à l'aide d'un agent alkylant difonctionnel, en particulier un halogénure d'alkylène avec, le cas échéant, deux molécules du tout-cis-1,3,5-triamino-2,4,6-cyclohexane-triol utilisé comme produit de départ à l'état de complexe de fer-(III) ou de chrome-(III), et pour préparer les diéthers de formule générale III selon la revendication 14, on effectue l'alkylation en partant d'un dérivé quaternisé de formule générale II.

28. Procédé selon l'une des revendications 24 à 27, caractérisé en ce que l'hydrogénation catalytique est effectuée sous une agitation intensive produisant des turbulences.

29. Procédé selon l'une des revendications 24, 25, 27 et 28, caractérisé en ce que l'alkylation du stade b) est realisée sous forme d'une alkylation réductrice à l'aide d'aldéhydes ou de cétones.

30. Procédé selon la revendication 29, caractérisé en ce que l'alkylation réductrice est réalisée soit à l'aide d'acide formique, soit, en solution aqueuse par $H_2/Pt$.

31. Procédé selon l'une des revendications 24 à 30, caractérisé en ce que l'alkylation du stade c) est réalisée à l'aide d'un halogénure d'alkyle, en particulier l'iodure de methyle.

32. Procédé selon l'une des revendications 24 à 31, caractérisé en ce que l'on utilise du trinitrophloroglucinol qui a été obtenu par
a) réaction du phloroglucinol avec l'anhydride acétique et
b) nitration du triacétate ainsi obtenu par l'acide nitrique fumant.